(19) Europäisches Patentamt
European Patent Office
Office européen des brevets

(11) EP 4 311 546 A1

(12) **EUROPEAN PATENT APPLICATION**

(43) Date of publication:
**31.01.2024 Bulletin 2024/05**

(21) Application number: **22306112.8**

(22) Date of filing: **26.07.2022**

(51) International Patent Classification (IPC):
***A61K 31/138*** *(2006.01)* ***A61K 45/06*** *(2006.01)*
***C12N 15/113*** *(2010.01)* ***A61P 21/00*** *(2006.01)*

(52) Cooperative Patent Classification (CPC):
(C-Sets available)
**A61K 31/138; A61K 45/06; A61P 21/00;**
**C12N 15/1137; C12Y 306/05005;** C12N 2310/11;
C12N 2310/315; C12N 2310/344; C12N 2320/31

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Dynacure**
**67400 Illkirch-Graffenstaden (FR)**

(72) Inventors:
- **COWLING, Belinda**
**Illkirch-Graffenstaden (FR)**
- **THIELEMANS, Leen**
**Illkirch-Graffenstaden (FR)**
- **PERDOMINI, Morgane**
**Illkirch-Graffenstaden (FR)**
- **COLOMBO, Sophie**
**Illkirch-Graffenstaden (FR)**

(74) Representative: **Cabinet Becker et Associés**
**25, rue Louis le Grand**
**75002 Paris (FR)**

Remarks:
The complete document including Reference Table(s) and the Sequence Listing(s) can be downloaded from the EPO website

(54) **COMBINATION THERAPY FOR MYOPATHIES**

(57) The present invention relates to a new approach for treating a myopathy selected from a dystrophinopathy or a centronuclear myopathy (CNM). More particularly, the present invention relates to a combination comprising: (i) a dynamin 2 inhibitor; and (ii) a compound of formula (I) or an analog or a metabolite thereof, or a pharmaceutically acceptable salt, solvate, oxide, or stereoisomer thereof, for use in treating said myopathy in a subject in need thereof.

EP 4 311 546 A1

(52) Cooperative Patent Classification (CPC): (Cont.)

C-Sets
**A61K 31/138, A61K 2300/00;**
C12N 2310/344, C12N 2310/3231

## Description

## INTRODUCTION

The present invention relates to a new approach for treating a myopathy selected from a centronuclear myopathy (CNM) or a dystrophinopathy. More particularly, the present invention relates to a combination comprising: (i) a dynamin 2 inhibitor; and (ii) a compound of formula (I) or an analog or a metabolite thereof, or a pharmaceutically acceptable salt, solvate, oxide, or stereoisomer thereof, for use in treating said myopathy in a subject in need thereof.

Myopathies are a heterogeneous group of disorders primarily affecting the skeletal muscle structure, metabolism or channel function. They usually present with muscle weakness interfering in daily life activities, muscle pain being also a common finding. The etiology of myopathies is usually caused by a disruption in the muscle tissue integrity, and the metabolic stability which may be triggered by either inherited genetic diseases, or metabolic errors, certain drugs and toxins, bacterial or viral infections, inflammation, besides minerals, electrolytes, and hormonal irregularities.

Among myopathies, centronuclear myopathy belongs to a rare subgroup of congenital muscle disorders that are characterized by a general disorganization of myofibers, with abnormal positioning of different organelles, internal membrane alterations and often myofibrillar misalignment and fiber shape defects. A key hallmark is the localization of cell nuclei the center of the muscle fibers rather than at the edge, in the absence of increased muscle regeneration commonly observed in dystrophies. Yet, these disorders are clinically and genetically heterogeneous. Indeed, although most patients present with muscle weakness and low muscle tone, onset of symptoms and severity ranges from neonatal onset of severe weakness requiring respiratory and feeding intervention to onset of mild weakness in adulthood requiring assistance with mobility. The clinical presentation can appear similar to congenital or limb girdle muscular dystrophy, but the creatine kinase level is generally not elevated and muscle biopsies do not show significant dystrophic features. This is why diagnosis of centronuclear myopathy is essentially based on the presence of increased central nuclei in muscle biopsy. Genetic testing can then be used to discriminate between different subtypes of centronuclear myopathy, which are essentially referred as X-linked centronuclear myopathy (XL-CNM), autosomal recessive centronuclear myopathy (AR-CNM), and autosomal dominant centronuclear myopathy (AD-CNM). XL-CNM is the most common and severe form of CNM, with a neonatal onset presenting with weakness and secondary respiratory and feeding issues that often require support, and with a poor prognosis in about 60% of patients. Other CNM are extremely rare, AD-CNM tending to have a less severe phenotype than the autosomal recessive version.

Muscular dystrophy represents another subgroup of myopathies, that are hereditary, degenerative disorders characterized by progressive degeneration of muscle fibres and muscle tissue over time finally leading to progressive muscle weakness and premature death. Classical histological findings of muscular dystrophies consist of pathological fibre size variation, muscle cell degeneration (necrosis) and regeneration, and replacement of muscle by connective and adipose tissue.

Muscular dystrophies include, *inter alia,* dystrophinopathies such as Duchenne muscular dystrophy (DMD) and Becker muscular dystrophy (BMD), which are both caused by mutations in the dystrophin *DMD* gene. Depending upon the preservation or not of the reading frame, dystrophin can be either completely absent in DMD, or present in either a mutated or a truncated form in BMD. Due to the total loss of dystrophin, the first clinical signs of Duchenne muscular dystrophy (DMD) are severe and appear at an early age, generally around 2-5 years of age. Children affected by DMD, which are mostly boys, typically encounter difficulties first to walk and climb, then to run and breath, and will become wheelchair bound by 12-16 years old of age. These clinical signs, also accompanied by elevated creatine kinase levels, are due to a progressive muscle wasting and weakness affecting almost all muscles, which will ultimately result in a premature death from cardiorespiratory failure. Becker muscular dystrophy (BMD) is characterized by similar clinical signs, albeit with a very variable time course and severity. Some BMD patients are relatively asymptomatic, while others may become be wheelchair-dependent early on. This means that some will survive until an advanced age, while others will have a premature death. The expression of mutated dystrophin can be observed in BMD muscles, with a high variable extent ranging from less than 10% to 70% of the full-length expression of healthy muscles.

Effective therapies are required for these debilitating myopathies which improve overall muscle function and prevent or delay the onset and/or disease progression. To this day, despite the great progress made in research into these therapies and their ongoing development, no transformative therapy has yet been clinically approved for these diseases (Gómez-Oca et al., Int J Mol Sci., 2021; 22(21):11377; Al Zaidi et al., Pediatr Neurol., 2014; 51(5):607-618)

The present Inventors are herein the first to investigate an innovative approach aimed at (i) reducing in DNM2 expression, activity or function in combination with (ii) a small molecule known for its capacity to improve muscle strength, so as to treat the above-mentioned diseases. To do so, they assessed the efficacy of this proof-of-concept in an animal model of a myotubular myopathy, using an antisense targeting *Dnm2* mRNA in combination with tamoxifen. While Dynamin 2 has been previously identified as a therapeutic target to CNM (WO 2015/055859 A1; Cowling et al., J Clin Invest., 2014;124(3):1350-63; Tasfaout et al., Nat Commun. 2017; 8: 15661; Buono et al., Proc Natl Acad Sci U S A, 2018; 115(43):11066-11071; Trochet et al., EMBO Mol Med., 2018; 10(2): 239-253) as well as DMD (WO 2016/170162

A1), the inhibition of this particular target combined with tamoxifen had never been studied.

Unexpectedly, this combination produced a synergistic improvement, thereby confirming the suitability of this approach for treating not only a centronuclear myopathy but also a dystrophinopathy. In addition to prolonging lifespan, said combination improved body weight, restored whole-body strength, and significantly reduced the Disease Severity Score and hence delayed disease progression. These results are all the more surprising given that such effects were obtained with relatively low doses of each agent. It should nevertheless be understood that the combination of (i) any dynamin 2 inhibitor with (ii) any compound of formula (I) or an analog or a metabolite thereof, or a pharmaceutically acceptable salt, solvate, oxide or stereoisomer thereof, would achieve the same effect.

## SUMMARY OF THE INVENTION

In a first aspect, the invention relates to a combination for use in the treatment of a myopathy selected from a dystrophinopathy or a centronuclear myopathy (CNM), the combination therapy comprising:

(i) a dynamin 2 inhibitor; and

(ii) a compound of formula (I), or an analog or a metabolite thereof,

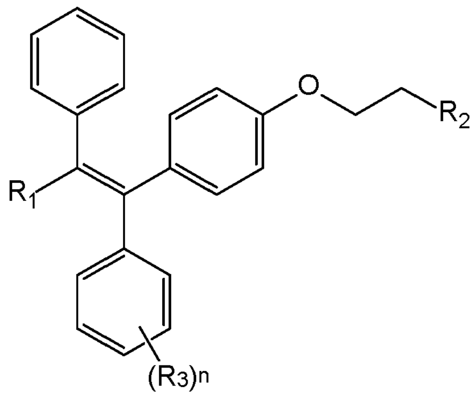

(I),

wherein

- $R_1$ is a radical selected from:
  - a ($C_1$-$C_6$)alkyl group optionally substituted by a halogen,
  - a hydrogen, or
  - a halogen;
- $R_2$ is a radical selected from:
  - a -$NR_4R_5$ group with $R_4$ and $R_5$ being independently a hydrogen, a ($C_1$-$C_6$)alkyl group said ($C_1$-$C_6$)alkyl group being optionally substituted by a -$CF_3$ or a -$OCF_3$ group, or $R_4$ and $R_5$ form together a heterocycloalkyl, or
  - a -$OR_6$ group with $R_6$ being a H or a ($C_1$-$C_6$)alkyl group substituted by a hydroxy;
- n is 0, 1, or 2; and/or
- $R_3$ is a radical selected from:
  - a hydrogen,
  - a hydroxy, or
  - a halogen, preferably a iodine;

or a pharmaceutically acceptable salt, solvate, oxide, or stereoisomer thereof.

In a preferred embodiment, the dystrophinopathy is Duchenne muscular dystrophy or Becker muscular dystrophy, preferably Duchenne muscular dystrophy.

In a preferred embodiment, the centronuclear myopathy is X-linked centronuclear myopathy, autosomal recessive centronuclear myopathy or autosomal dominant centronuclear myopathy, preferably X-linked centronuclear myopathy.

In a preferred embodiment, the dynamin 2 inhibitor is selected from the group consisting of a nucleic acid molecule interfering specifically with dynamin 2 expression, an antibody directed against dynamin 2, a genome editing system comprising a nuclease engineered to target the *DNM2* gene, and a small molecule inhibiting the dynamin 2 activity, expression or function, and any combinations thereof.

In a preferred embodiment, the nucleic acid molecule interfering specifically with dynamin 2 expression is an antisense nucleic acid, an RNAi nucleic acid, or a ribozyme interfering specifically with dynamin 2 expression.

In a preferred embodiment, the nucleic acid molecule interfering specifically with dynamin 2 expression is complementary to at least one intron or the 3'UTR of dynamin 2 pre-mRNA, preferably to at least intron 12, intron 13, intron 11, intron 1, intron 14 of dynamin 2 pre-mRNA.

In a preferred embodiment, the nucleic acid molecule interfering specifically with dynamin 2 expression is an antisense nucleic acid comprising or consisting of at least 8 contiguous nucleobases of any one of SEQ ID NO: 1 to 3135, preferably is antisense nucleic acid comprising or consisting of any one of the sequences of SEQ ID NO: 1 to 3135.

In a preferred embodiment, the nucleic acid molecule interfering specifically with dynamin 2 expression induces exon-skipping within a dynamin 2 pre-mRNA, preferably of at least exon 2 and/or 8 of dynamin 2 pre-mRNA.

In a preferred embodiment, the nucleic acid molecule interfering specifically with dynamin 2 expression is an antisense nucleic acid comprising or consisting of the sequence SEQ ID NO: 3136 or SEQ ID NO: 3137.

In a preferred embodiment, the nucleic acid molecule interfering specifically with dynamin 2 expression is complementary to at least one exon of dynamin 2 mRNA, preferably to at least exon 1, 4, 5, 12b, 13, 15, 17 and/or 21 of dynamin 2 mRNA.

In a preferred embodiment, the nucleic acid molecule interfering specifically with dynamin 2 expression is an RNAi nucleic acid comprising or consisting of any one of the sequences SEQ ID NO: 3138 to SEQ ID NO: 3151.

In a preferred embodiment, the compound of formula (I) is such that:

- $R_1$ is a radical selected from:
  - ◦ a $(C_1\text{-}C_6)$alkyl group, preferably an ethyl group, optionally substituted by a halogen, preferably a chlorine; or
  - ◦ a halogen, preferably a chlorine;
- $R_2$ is a radical selected from:
  - ◦ a $-NR_4R_5$ group with $R_4$ and $R_5$ being independently a hydrogen, a $(C_1\text{-}C_6)$alkyl group, preferably a methyl or an ethyl group, or $R_4$ and $R_5$ form together a pyrrolidinyl; or
  - ◦ a $-OR_6$ group with $R_6$ being a H or a $(C_1\text{-}C_6)$alkyl group, preferably an ethyl group, substituted by a hydroxy;
- n is 0 or 1 ; and/or
- $R_3$ is a radical selected from: a hydrogen, a hydroxy, or a halogen, preferably a iodine.

In a preferred embodiment, the compound of formula (I) is such that $R_1$ is an ethyl group, $R_2$ is a $NR_4R_5$ group with $R_4$ and $R_5$ being independently a methyl group, and n is 0.

In a preferred embodiment, the analogs of the compound of formula (I) are selected from the group consisting of:

- 6-(2,4-dichlorophenyl)-5-[4-[(3S)-1-(3-fluoropropyl)pyrrolidin-3-yl]oxyphenyl]-8,9-dihydro-7H-benzo[7]annulene-2-carboxylic acid;
- (SR,6S)-6-phenyl-5-[4-(2-pyrrolidin-1-ylethoxy)phenyl]-5,6,7,8-tetrahydronaphthalen-2-ol;
- [6-hydroxy-2-(4-hydroxyphenyl)-1-benzothiophen-3-yl]-[4-(2-piperidin-1-ylethoxy)phenyl]methanone;
- 1-[[4-[2-(azepan-1-yl)ethoxy]phenyl]methyl]-2-(4-hydroxyphenyl)-3-methylindol-5 -ol;
- (E)-4-[2-[4-[(E)-1-(1H-indazol-5-yl)-2-phenylbut-1enyl]phenoxy]ethylamino]-N,N-dimethylbut-2-enamide; and
- (E)-3-[4-[(E)-2-(2-chloro-4-fluorophenyl)-1-(1H-indazol-5-yl)but-1-enyl]phenyl]prop-2-enoic acid.

In a further aspect, the invention relates to (i) a dynamin 2 inhibitor and (ii) a compound of formula (I) or an analog or a metabolite thereof, or a pharmaceutically acceptable salt, solvate, oxide, or stereoisomer thereof, as described herein, as a combined preparation for simultaneous, separate or sequential use in the treatment of a myopathy of the invention.

In another aspect, the invention relates to a pharmaceutical composition comprising (i) a dynamin 2 inhibitor and (ii) a compound of formula (I) or an analog or a metabolite thereof, or a pharmaceutically acceptable salt, solvate,

oxide, or stereoisomer thereof, as described herein, and optionally (iii) a pharmaceutically acceptable excipient.

## LEGENDS TO THE FIGURES

**Figure 1. Lifespan of mice is improved with the combination therapy according to the invention**. Kaplan-Meier representation of survival (percentage) of WT and *Mtm1*$^{-/y}$ mice injected weekly from 4 weeks old with 3 mg/kg of ASO-ctrl (control ASO), or ASO-Dnm2 to 12 weeks of age and/or treated with tamoxifen at 10 mg/kg of diet (n = 10 mice per group). A log-rank (Mantel-Cox) test was performed between *Mtm1*$^{-/y}$ ASO-Dnm2 and *Mtm1*$^{-/y}$ Tamoxifen groups, between *Mtm1*$^{-/y}$ ASO-Dnm2 and *Mtm1*$^{-/y}$ ASO-Dnm2 + Tam groups and between *Mtm1*$^{-/y}$ Tamoxifen and *Mtm1*$^{-/y}$ ASO-Dnm2 + Tam groups (non-significant).

**Figure 2. Body weight of mice is improved with the combination therapy according to the invention**. (A) Evolution of body weight from 3 weeks of age (before treatment) to 12 weeks of age (before sacrifice), and (B) body weight at 12 weeks old, in *Mtm1*$^{-/y}$ and WT mice injected weekly from 4 weeks old with 3 mg/kg of ASO-ctrl (control ASO) or ASO-Dnm2 to 12 weeks of age and/or treated with tamoxifen at 10 mg/kg of diet (n = 10 mice per group). Mean ± 95% CI is represented for body weight evolution graph. A one-way ANOVA followed by a Sidak's multiple comparisons *post-hoc* test was performed on 12 weeks old data. P-value: **** < 0.0001. *Mtm1*$^{-/y}$ mice injected with ASO-ctrl and represented with black rhombi were sacrificed for humane reasons or died prior to 13 weeks of age, and therefore could not be used for statistical analysis and direct comparisons.

**Figure 3. Muscle strength is improved with the combination therapy according to the invention. (A)** Evolution of whole-body strength (hanging test) from 4 weeks of age (before first dose) until 12 weeks of age (after last dose), and **(B)** whole-body strength at 12 weeks old, in *Mtm1*$^{-/y}$ and WT mice injected weekly from 4 weeks old with 3 mg/kg of ASO-ctrl (control ASO), or ASO-Dnm2 to 12 weeks of age and/or treated with tamoxifen at 10 mg/kg of diet (n =10 mice per group). Mean ± 95% CI is represented for hanging time evolution graph. A one-way ANOVA was performed followed by a Sidak's multiple comparison *post-hoc* test was performed on 12 weeks old data. Data were transformed using the function Y = Y/K with K = 60.1 and Y = logit(Y). P-value: **< 0.01. *Mtm1*$^{-/y}$ mice injected with ASO-ctrl and represented with black rhombi were sacrificed for humane reasons or died prior to 13 weeks of age, and therefore could not be used for statistical analysis and direct comparisons.

**Figure 4. Disease severity score is improved with the combination therapy according to the invention. (A)** Evolution of total Disease Severity Score (DSS) from 4 weeks of age (before first dose) to 12 weeks of age (after last dose), and **(B)** DSS at 12 weeks old in *Mtm1*$^{-/y}$ mice and WT injected weekly from 4 weeks old with 3 mg/kg of ASO-ctrl (control ASO) or ASO-Dnm2 to 12 weeks of age and/or treated with tamoxifen at 10 mg/kg of diet (n = 10 mice per group). Mean ± 95% CI is represented for DSS evolution graph. A one-way ANOVA (residuals were normally distributed and standard deviations not statistically different) was performed followed by a Sidak's multiple comparison *post-hoc* test was performed on 12 weeks old data. Data were transformed using the function Y = Y/K with K = 5.1 and Y = logit(Y). P-values: * < 0.05, **< 0.01. *Mtm1*$^{-/y}$ mice injected with ASO-ctrl and represented with black rhombi were sacrificed for humane reasons or died prior to 13 weeks of age, and therefore could not be used for statistical analysis and direct comparisons.

**Figure 5. Tamoxifen has no effect on Dynamin 2 expression, whether (A) at the RNA level or (B) at the protein level. (A)** *Dnm2* mRNA expression in C2C12 cells (immortalized mouse myoblasts) after treatment with PBS (control), 2 μM ASO-ctrl or ASO-Dnm2 alone, 20 μM Tamoxifen alone, or the combination of ASO-ctrl or ASO-Dnm2 with Tamoxifen. Expression was normalized to the reference genes *Rpl27* and *Tbp.* (B) DNM2 protein expression in C2C12 cells after treatment with PBS (control) + 20 μM Tamoxifen, 2 μM ASO-ctrl or ASO-Dnm2 alone, or in combination with Tamoxifen at 5, 10 or 20 μM. Expression was normalized to GAPDH as loading control.

**Figure 6. Example of a preferred Dynamin 2 inhibitor according to the invention.**

## DETAILED DESCRIPTION OF THE INVENTION

Unless otherwise defined herein, scientific and technical terms used in connection with the present invention shall have the meanings that are commonly understood by those of ordinary skill in the art. Further, unless otherwise required by context, nomenclatures used herein, and techniques of molecular and cellular therapy are those well-known and commonly used in the art.

The present invention may be understood more readily by reference to the following detailed description, included preferred embodiments of the invention, and examples included herein.

The present invention aims at providing an innovative therapeutic approach for the treatment of a centronuclear myopathy (CNM) or a dystrophinopathy in a subject in need thereof by (i) reducing DNM2 expression, activity or function in combination in combination with (ii) a small molecule selected from tamoxifen and the like.

In a first aspect, the present invention relates to a combination for use in the treatment of a myopathy selected from a centronuclear myopathy or a dystrophinopathy, preferably a centronuclear myopathy, the combination comprising: (i) a dynamin 2 inhibitor; and (ii) a compound of formula (I) or an analog or a metabolite thereof, or a pharmaceutically acceptable salt, solvate, oxide, or stereoisomer thereof.

In particular, the present invention is directed to the combined use of (i) a dynamin 2 inhibitor; and (ii) a compound of formula (I) or an analog or a metabolite thereof, or a pharmaceutically acceptable salt, solvate, oxide, or stereoisomer thereof, for the preparation of a medicament for the treatment of a myopathy selected from a centronuclear myopathy or a dystrophinopathy, preferably a centronuclear myopathy.

Further provided is a method for treating a myopathy selected from a centronuclear myopathy or a dystrophinopathy, preferably a centronuclear myopathy, in a subject in need thereof, said method comprising the administration, to said subject, of a therapeutically effective amount of: (i) a dynamin 2 inhibitor; and (ii) a compound of formula (I) or an analog or a metabolite thereof, or a pharmaceutically acceptable salt, solvate, oxide, or stereoisomer thereof.

*"Centronuclear myopathy"* (CNM) is an umbrella term for a group of rare congenital myopathies characterized by an increased number of muscle fibers with nuclear centralization (hence, the centronuclear feature) in the absence of excessive regeneration (contrary to dystrophies), myofiber atrophy, predominance of type I myofibers, which translates clinically into muscle weakness and associated disabilities such as breathing difficulty. Three main forms of centronuclear myopathy have been identified to this day, which essentially differ by their mutation and/or mode of inheritance: X-linked centronuclear myopathy (XL-CNM, OMIM 310400); autosomal recessive centronuclear myopathy (AR-CNM, OMIM 255200); and autosomal dominant centronuclear myopathy (AD-CNM, OMIM 160150).

*"X-linked centronuclear myopathy"* (XL-CNM), also called "*myotubular myopathy*" (XL-MTM, XLMTM or X-MTM) is the most common (prevalence of about 60%) and severe form of CNM, mainly observed in males (1/50,000 newborn males each year), with neonatal and death often occurring in the first years of life (Jungbluth et al., Orphanet J Rare Dis, 2008; 3:26). XL-CNM is due to mutation in myotubularin 1 (MTM1) located on the X-chromosome (Laporte et al., Nature Genetics, 1996; 13(2): 175-182), and is typically characterized by marked muscle weakness, hypotonia as well as feeding and breathing difficulties. To date more than 200 different mutations in *MTM1* have been reported in about 450 families, most of which leads to a strong reduction in protein expression.

The autosomal dominant forms of centronuclear myopathy, referred herein as *"autosomal dominant CNM*" (AD-CNM), are mostly due to mutation in the gene encoding dynamin 2 (DNM2) (Bitoun et al., Nature Genetics, 2005; 37(11): 1207-1209). Without being bound by theory, it is thought that some genetic mutation in BIN1 (Bohm et al., Brain, 2014; 137(Pt 12):3160-3170), MYF6 (Kerst et al. Neuromuscul Disord., 2000; 10:572-577), MTMR14 (Tosch et al., Hum Mol Genet., 2006; 15(21):3098-3106) or CCDC78 (Majczenko et al, Am. J. Hum. Genet., 2012; 91: 365-371) gene can also be responsible for this phenotype. Among AD-CNM, DNM2-CNM is highly variable in presentation and severity: mild cases can go unnoticed for prolonged periods due to a late onset, typically in the thirties; while others can have symptoms that are present in infancy or early childhood with e.g. hypotonia, generalized weakness, facial muscle weakness, ptosis, and ophthalmoplegia.

*"Autosomal recessive centronuclear myopathy"* (AR-CNM) is caused by mutation in the gene (BIN1) encoding the membrane remodeling protein amphiphysin 2 (Nicot et al., Nature Genetics, 2007; 39(9): 1134-1139). Without being bound by theory, it is thought that biallelic mutation in RYR1 (encoding the ryanodine receptor), SPEG (encoding the striated muscle enriched protein kinase), TTN (encoding Titin), or ZAK (encoding a kinase; Valsi et al., Brain, 2017; 140: 37-48) gene can also be responsible for this phenotype , though these types of AR-CNM appear to be more sporadic.

X-linked centronuclear myopathy, autosomal recessive centronuclear myopathy, and autosomal dominant centronuclear myopathy are preferred centronuclear myopathies to be treated according to the invention. A more particularly preferred centronuclear myopathy to be treated according to the invention is X-linked centronuclear myopathy.

The terms "*dystrophinopathy ",* "*dystrophin-related myopathy", "muscular dystrophin-related disease",* or *"muscular dystrophinopathy"* are herein interchangeable, and refer to a myopathy characterized by muscular dystrophy and which is associated with a defect in muscle dystrophin. Said defect is typically caused by one or more mutations in the dystrophin gene; said pathology can accordingly be referred as a genetic muscular dystrophy. Duchenne (DMD) and Becker (BMD) muscular dystrophies are genetic muscular dystrophies with a relatively high incidence, which are caused by at least one mutation in the dystrophin gene. Phenotypic features of said pathologies are as described above.

In DMD, dystrophin is either absent or truncated non-functional, generally as a result of a frameshift mutation in the *DMD* gene, such as a frameshift mutation/out-of-frame deletion or duplication, or a nonsense mutation. Histologically, this mutation leads to alternating cycles of fiber necrosis and regeneration. Over time, the ongoing regeneration process is exhausted, and fibrosis and adipose tissue replacement of muscle fibers occurs. Lesions of the plasma

membrane can be revealed by electron microscopy, and the absence of dystrophin can be observed by immunoblotting or fluorescent labelling, except in some revertant fibers.

By contrast, and as a general rule (i.e. in the majority of patients), BMD is typically caused by a mutation that does not cause a frameshift in the *DMD* gene, thereby generating a partially functional dystrophin; these mutations may be in-frame deletions or duplications of one or more exons, or splice-site mutations. Nevertheless, nonsense and frameshifts mutations leading to some dystrophin with remaining activity have been identified in BMD

Mutations responsible for DMD and BMD have been extensively reviewed in the literature (Muntoni et al., Lancet Neurol, 2003, 2: 731-740; Tuffery-Giraud et al., Hum Mutat., 2009, 30(6):934-45; Bladen et al., Hum Mutat. 2015;36(4):395-402;).

Duchenne muscular dystrophy and Becker muscular dystrophy are particularly preferred dystrophin-related myopathies to be treated according to the invention. A more particularly preferred dystrophinopathy to be treated according to the invention is Duchenne muscular dystrophy.

Generally speaking, the term *"treatment"* or "*treating*" means obtaining a desired physiological or pharmacological effect depending on the degree of severity of the symptom or disorder of interest, or risks thereof, i.e. herein, depending on the degree of severity or risks of developing such symptom or disorder. The effect may be prophylactic in terms of a partial or complete prevention of the symptom or disorder and/or may be therapeutic in terms of a partial or complete cure of the symptom or disorder. The term *"prophylactic"* characterizes the capacity to avoid, or minimize the onset or development of a symptom or disorder before its onset (for example, before the onset of muscle weakness). The term *"therapeutic"* refers to the capacity to inhibit the symptom or disorder (i.e. arresting the development thereof), and/or to relieve said symptom or disorder (i.e. regression leading to an improvement). A prophylactic effect is generally said to be achieved when e.g. an asymptomatic subject remains asymptomatic or quasi-asymptomatic after treatment according the invention (for example, absence of muscle weakness and/or respiratory difficulties), while a therapeutic effect is typically said to be achieved when e.g. a symptomatic subject recovers, partially or totally, after treatment according to the invention (for example, partial or complete recovery of muscle strength and/or respiratory function).

It should be understood that even a partial or intermittent relief of at least one symptom of the centronuclear myopathy or dystrophinopathy can be of great benefit for the patient, and is thus considered herein as an effective treatment. In addition, treatment of the patient may be a single event, or the patient is administered with the combination according to the invention on multiple occasions, that may be, depending on the results obtained, several days apart, several weeks apart, or several months apart, or even several years apart. A treatment according to the invention may have a duration of at least one week, at least one month, at least several months, at least one year, at least 2, 3, 4, 5, 6 years or more. The frequency of administration may range between at least once every week, two weeks, three weeks, four weeks or five weeks or a longer time period.

A *"therapeutically effective amount*" means herein an amount that is sufficient to achieve the effect for which it is indicated, herein the treatment of a centronuclear myopathy or a dystrophinopathy. The amount of the combination according to the invention to be administered can be determined by standard procedures well known by those of ordinary skill in the art. Physiological data of the patient (e.g. age, size, and weight), the routes of administration and the disease to be treated have to be taken into account to determine the appropriate dosage, optionally compared with subjects that do not suffer from a centronuclear myopathy or dystrophinopathy. The amount may also vary according to other components of a treatment protocol (e.g. administration of other medicaments, etc.).

It should be further understood that the "*subject*" or *"host"* to be treated according to the invention is one having a muscular system. Accordingly, preferred subjects to be treated are animals, more preferably mammals, most preferably humans.

The term *"dynamin 2"* (DNM2), also known as CMTDI1, CMTDIB, DI-CMTB, DYN2, dynamin II, or DYNII, refers to a GTPase protein involved in endocytosis, membrane trafficking and fission, and cytoskeleton organization. The dynamin 2 protein contain 5 domains: an N-terminal GTPase domain (G-domain) that binds and hydrolyzes GTP; a middle domain enabling dimerization of the protein; a pleckstrin homology (PH) domain promoting binding to phosphoinositides; a GTPase effector domain (GED) and a C-terminal proline-rich domain (PRD) involved in protein-protein interactions. In humans, dynamin 2 is encoded by the *DNM2* gene located on chromosome 19 and composed of 22 exons (genomic DNA sequence available on the NCBI database under the reference sequence NG_008792.1). The human DNM2 protein exists in five alternatively spliced variants: DNM2 isoform 1 (amino acid sequence: NP_001005360.1, coded by mRNA sequence: NM_001005360.3); DNM2 isoform 2 (amino acid sequence: NP_001005361.1, coded by mRNA sequence: NM_001005361.3); DNM2 isoform 3 (amino acid sequence: NP_004936.2, coded by RNA sequence : NM_004945.4); DNM2 isoform 4 (amino acid sequence: NP_001005362.1, coded by RNA sequence : NM_001005362.3); and DNM2 isoform 5 (amino acid sequence: NP_001177645.1, coded by RNA sequence : NM_001190716.2).

As used herein, a "*dynamin-2 inhibitor*" refers to any molecule capable of decreasing specifically the expression of dynamin 2 or inhibit the dynamin 2 activity or function, either directly or indirectly. Preferably, the dynamin 2 inhibitor is a direct inhibitor, meaning that it interacts directly with either the dynamin 2 protein or a nucleic acid encoding said

dynamin 2 or a part thereof. The dynamin 2 inhibitors according to the invention are capable of inhibiting or decreasing the functional activity of dynamin 2 *in vivo* and/or *in vitro.* The inhibitor may inhibit the functional activity of dynamin 2 by at least about 10%, 20%, preferably by at least about 30%, 40%, preferably by at least about 50%, 60%, preferably by at least about 70% or 80%. In particular, the inhibitor may inhibit dynamin 2 expression by at least about 10%, 15%, preferably by at least about 20%, 25%, preferably by at least about 30%, 35%, preferably by at least about 40%, 45%, preferably by at least about 50%, 55%, preferably by at least about 60%, 65%. More particularly, the inhibitor may inhibit dynamin 2 expression by at least about 15%, preferably by at least about 20%, 25%, preferably by at least about 30%, 35%, preferably by at least about 40%, 45%, preferably by at least about 50%, 55%, preferably by at least about 60%.

A dynamin 2 inhibitor of the invention may act by blocking and/or inhibiting the activity or function of dynamin 2. This may for example be achieved by inhibiting the enzymatic activity of dynamin 2. Functional or enzymatic activity of dynamin 2 may be readily assessed by one skilled in the art according to known methods by testing, for example, the GTPase activity or the function of dynamin 2 in clathrin-mediated endocytosis (Macia E. et al, Developmental cell 2006; 10, 839-850). For inhibitors of GTPase activity or lipid binding, subcellular localization, clathrin mediated endocytosis, synaptic vesicle endocytosis, one can use the method described in McCluskey et al. (Traffic, 2013;14(12):1272-89), or McGeachie et al. (ACS Chem Biol, 2013 ;8(7):1507-18). For dynamin 2 GTPase activity, oligomerisation, lipid binding, one can use the method described by Wang et al. (J Biol Chem, 2010; 285(30):22753-7), or by Kenniston and Lemmon (EMBO J, 2010; 29(18):3054-67).

The dynamin 2 inhibitor of the invention may also act by blocking and/or inhibiting the dynamin 2 expression (including transcription, splicing, transcript maturation, or translation). The decrease or inhibition of dynamin 2 expression can be evaluated by any means known to those skilled in the art including, but not limited to, assessing the level of Dynamin 2 protein using for instance Western Blot or ELISA with a specific anti-Dynamin 2 antibody, and/or assessing the level of mRNA transcript for Dynamin 2 using e.g. quantitative PCR.

Dynamin 2 inhibitors have been reported in the scientific and patent literature, and are thus well-known in the art (WO2015055859; WO2018/00010; WO2018189208; WO2019140452; WO2020028844; Tasfaout et al., Nat Commun 2017, 8: 15661; Buono et al., Proc Natl Acad Sci USA 2018, 115(43): 11066-11071; the contents of which are incorporated herein by reference in their entireties). Below is provided a non-exhaustive list of such inhibitors which can be used in the present invention.

The dynamin 2 inhibitor used in the present invention is preferably selected from the group consisting of a nucleic acid molecule interfering specifically with dynamin 2 expression, an antibody directed against dynamin 2, a genome editing system comprising a nuclease and at least one *DNM2* guide RNA, a small molecule inhibiting the dynamin 2 enzymatic activity, expression, or function, and any combinations thereof.

In a preferred embodiment, the dynamin 2 inhibitor used in the present invention is a nucleic acid molecule interfering specifically with dynamin 2 expression.

As used throughout the specification, the terms *"nucleic acid molecule",* "*nucleic acid*", *"polynucleotide"* (polynucleoside) or *"oligonucleotide"* (oligonucleoside) refers to a succession of natural or synthetic nucleotides (or nucleosides) connected by internucleotide (or internucleoside) linkages, wherein each nucleotide (or nucleoside) or internucleotide (or internucleoside) linkages may be modified or unmodified. A nucleotide is comprised of a nucleoside and a phosphate group. A nucleoside is comprised of a nucleobase and a sugar. A nucleobase comprises a modified and unmodified nucleobase. Unmodified nucleobases are well-known in the art and include adenine (A), thymine (T), cytosine (C), uracil (U) and guanine (G). An internucleotide (internucleoside) linkage is a bond that forms a covalent linkage between adjacent nucleotides (nucleosides) in a nucleic acid, such as phosphate internucleotide linkages that are naturally occurring. A nucleic acid may be a single-stranded or double-stranded DNA such as cDNA, genomic DNA, ribosomal DNA, and the transcription product of said DNA, such as RNA. Nucleic acids also encompass nucleic acids encoding a peptide or polypeptide of interest, as well as nucleic acids which can hybridize to a nucleic acid of reference. When a nucleic acid is designed to hybridize to a nucleic acid of reference, it can be chemically modified to enhance its stability, nuclease resistance, target specificity and/or improve their pharmacological properties (e.g. reduced toxicity, increased intracellular transport, etc.). For example, a nucleic acid may comprise modified nucleotide(s) and/or backbone, such as a modified sugar, a modified nucleobase, and/or a modified internucleotide (internucleoside) linkage. A standard modification is the replacement of native phosphates of the internucleotide (internucleoside) linkage with phosphorothioates (PS) so as to reduce the sensitivity to nucleases and accordingly improve the stability, half-life and tissue distribution of the nucleic acid (Eckstein F., Antisense and Nucleic Acid Drug Development, 2000, 10(2): 1117-221). Another standard modification which increases the affinity of the nucleic acid towards its target is the incorporation in the sugar moiety of a methoxyethyl (MOE) or a constrained ethyl (cEt) to the 2'position, or the tethering of the 4'-carbon to the 2-hydroxyl of the ribose ring to create a bicyclic locked nucleic acid (LNA), to name a few.

Preferred nucleic acid molecules interfering specifically with dynamin 2 expression according to the invention are those capable of hybridizing specifically to the gene or transcripts encoding dynamin 2, at least to a part thereof; as such, these are usually non-naturally occurring nucleic acids (i.e. synthetic).

"*Hybridizing*" or *"hydridization"* means the pairing or annealing to a target, herein under physiological conditions,

typically via hydrogen bonding between complementary nucleotides, such as Watson-Crick, Hoogsteen or reversed Hoogsteen hydrogen bonding. A specific hybridization therefore means that the pairing or annealing is specific to the target (no off-targets effects, or at least no substantial off-targets effects).

*"A nucleic acid molecule capable of hybridizing"* to a target nucleic acid thus means that a stretch of this nucleic acid is capable of forming base pairs to another stretch of the target nucleic acid. It is thus not absolutely required that all the bases in the region of complementarity are capable of pairing with bases in the opposing strand. Mismatches may be tolerated to some extent, as long as in the circumstances, the stretch of nucleotides is capable of hybridizing to its complementary part.

The nucleic acid molecule interfering specifically with dynamin 2 expression is preferably an antisense nucleic acid, an RNAi nucleic acid, or a ribozyme interfering specifically with dynamin 2 expression.

The term *"antisense nucleic acid*" or *"antisense oligonucleotides"* (ASO) designates a synthetic single-stranded oligonucleotide of which the sequence is at least partially complementary to a target nucleic acid, such as to a pre-mRNA or a mRNA sequence of a target gene (Lee et al., J Cardiovasc Transl Res. 2013; 6(6):969-80.; DeVos et al., Neurotherapeutics 2013; 10(3):486-972013). An antisense nucleic acid is capable of altering the expression of a specific target gene, either by splicing modification such as by inducing exon-skipping, or by recruiting RNAse H leading to RNA degradation of RNA-DNA duplex, thus blocking the expression of the target gene. An antisense nucleic acid is typically short in length, in general 5 to 50 nucleotides in length, such as 5, 10, 15, 20, 25, 30, 35, 40, 45 or 50 nucleotides in length, more preferably 15, 20, 25, 30, 35 nucleotides in length. It is well within the skill of the person in the art to design antisense nucleic acids specific to a target, based on the knowledge of a target sequence such as introns, exons, 5'CAP or pre-mRNA sequences (DeVos et al., Neurotherapeutics 2013; 10(3):486-972013). Antisense nucleic acids can be prepared by methods well-known in the art, such as by chemical synthesis and enzymatic ligation reactions.

In the context of the present invention, an antisense nucleic acid can be used to down-regulate the expression of dynamin 2. Said antisense nucleic acid can be complementary to all or part of a sense nucleic acid encoding dynamin 2, e.g., complementary to the coding strand of a double-stranded cDNA molecule or complementary to an mRNA sequence, and it is thought to interfere with the translation of the target mRNA.

In a preferred embodiment, the nucleic acid molecule interfering specifically with dynamin 2 expression is an antisense nucleic acid that is complementary to at least a part of the mRNA or pre-mRNA encoding dynamin 2.

For example, the nucleic acid molecule interfering specifically with dynamin 2 expression can be an antisense nucleic acid that is complementary to at least one intron or to the untranslated region (UTR) of the pre-mRNA encoding dynamin 2. The targeted region can be entirely within an intron or UTR of the *DNM2* pre-mRNA, or span an intron/exon junction, or cover at least 50% within an intron of the *DNM2* pre-mRNA.

In a preferred embodiment, the nucleic acid molecule interfering specifically with dynamin 2 expression is an antisense nucleic acid complementary to at least one intron or the 3'UTR of dynamin 2 pre-mRNA, preferably to at least one intron of dynamin 2 pre-mRNA.

In a preferred embodiment the nucleic acid molecule interfering specifically with dynamin 2 expression is an antisense nucleic acid comprising or consisting of at least 8 (preferably at least 8 to 12) contiguous nucleobases of any one of SEQ ID NO: 1 to 3128, even more preferably comprises or consists of any one of SEQ ID NO: 1 to 3128.

In a more preferred embodiment, the nucleic acid molecule interfering specifically with dynamin 2 expression is an antisense nucleic acid complementary to at least intron 12, intron 13, intron 11, intron 1, or intron 14 of dynamin 2 pre-mRNA of dynamin 2 pre-mRNA, more preferably is an antisense nucleic acid comprising or consisting of at least 8 to 16 contiguous nucleobases of any one of the sequences SEQ ID NO: 2873, SEQ ID NO: 3050, SEQ ID NO: 2117, SEQ ID NO: 2183, SEQ ID NO: 2447, SEQ ID NO: 2154 or SEQ ID NO: 2226, even more preferably comprises, or consists, of any one of the sequences SEQ ID NO: 2873, SEQ ID NO: 3050, SEQ ID NO: 2117, SEQ ID NO: 2183, SEQ ID NO: 2447, SEQ ID NO: 2154 or SEQ ID NO: 2226. As reported by WO2020028844 (incorporated by reference in its entirety), the latter antisense nucleic acids exhibit the best properties among SEQ ID NO: 1 to 3128.

Yet, in a more preferred embodiment, the nucleic acid molecule interfering specifically with dynamin 2 expression is an antisense nucleic acid complementary to at least intron 12 of dynamin 2 pre-mRNA, more preferably is an antisense nucleic acid comprising or consisting of at least 8 to 16 contiguous nucleobases of the sequence SEQ ID NO: 2873 or SEQ ID NO: 2117, even more preferably comprises, or consists, of the sequence SEQ ID NO: 2873 or SEQ ID NO: 2117.

Still, in an even more preferred embodiment, the nucleic acid molecule interfering specifically with dynamin 2 expression is an antisense nucleic acid complementary to at least intron 12 of dynamin 2 pre-mRNA, more preferably is an antisense nucleic acid comprising or consisting of at least 8, 9, 10, 11, 12, 13, 14, 15, 16 contiguous nucleobases of the sequence SEQ ID NO: 2873 even more preferably comprises, or consists, of the sequence SEQ ID NO: 2117.

As described above, the antisense nucleic acid may further comprise at least one modified sugar, at least one modified internucleotide (or internucleoside) linkage, and/or at least one modified nucleobase, so as to enhance its properties, such a reduction in sensitivity to nucleases, increase in stability, half-life and/or tissue distribution, and/or enhancement of its affinity towards its target nucleic acid.

In a preferred embodiment, the nucleic acid molecule interfering specifically with dynamin 2 expression is an

antisense nucleic acid, as described herein, that comprises at least one modified sugar, in particular in position 2' of the nucleotide (or nucleoside) such as 2'-O-methyl (2'-O-Me), 2'fluoro (2'-F), 2'O-methoxyethyl (2'-MOE) and 2',4'-brigded.

Particularly preferred modified sugars are bicyclic sugars, such as a cEt (constrained ethyl) bicyclic sugar, an LNA bicyclic sugar, or an ENA bicyclic sugar.

*"Bicyclic sugar"* or *"bicyclic sugar moiety"* means a modified sugar comprising two rings, wherein the second ring is formed via a bridge connecting two of the atoms in the first ring thereby forming a bicyclic structure.

A particularly preferred modified sugar according to the invention is cEt bicyclic sugar (constrained ethyl bicyclic sugar). This modified sugar is a bicyclic sugar moiety, wherein the first ring of the bicyclic sugar moiety is a ribosyl sugar moiety, the second ring of the bicyclic sugar is formed via a bridge connecting the 4'-carbon and the 2'-carbon (2',4'-brigded), the bridge has the formula 4'-$CH(CH_3)$-0-2', and the methyl group of the bridge is in the S configuration. A cEt bicyclic sugar moiety is in the β-D configuration.

In a preferred embodiment, the nucleic acid molecule interfering specifically with dynamin 2 expression is an antisense nucleic acid, as described herein, that comprises at least one modified internucleotide (or internucleoside) linkage, such as a phosphorothioate internucleotide (or internucleoside) linkage.

A *"modified internucleotide linkage"* (or modified internucleoside linkage) is an internucleotide (or internucleoside) linkage in a nucleic acid that is non-naturally occurring, and is thus referred as a non-phosphate linkage. Phosphorothioate linkage is a modified phosphate linkage in which one of the non-bridging oxygen atoms is replaced with a sulfur atom.

In a preferred embodiment, the nucleic acid molecule interfering specifically with dynamin 2 expression is an antisense nucleic acid, as described herein, that comprises at least one modified nucleobase, such as 5-methylcytosine (cytosine is methylated in 5' position).

In a preferred embodiment, the nucleic acid molecule interfering specifically with dynamin 2 expression is an antisense nucleic acid, as described herein, that is a gapmer.

A *"gapmer"* refers to a nucleic acid molecule comprising an internal segment having a plurality of nucleotides (or nucleosides) that support RNase H cleavage positioned between external segments, each having one or more nucleotides (or nucleosides), wherein the nucleotide (or nucleosides) comprising the internal segment are chemically distinct from the immediately adjacent nucleotide(s) (or nucleoside(s)) comprising the external segments. The internal or central segment may be referred to as the *"gap"* or *"gap segment"* and the external segments may be referred to as the *"wings"* or *"wing segments".* Typically, such gapmer can comprise a gap segment of 5 to 15 deoxynucleotides (or deoxynucleosides) flanked by wing segments of 2 to 10 modified nucleotides (or nucleosides).

For example, the nucleic acid molecule interfering specifically with dynamin 2 expression is an antisense nucleic acid, as described herein, that is a gapmer comprising:

a gap segment consisting of linked 2'-deoxynucleotides (or 2'-deoxynucleosides),
a 5' wing segment consisting of linked nucleotides (or nucleosides), and
a 3' wing segment consisting of linked nucleotides (or nucleosides),

wherein the gap segment is positioned between the 5' wing segment and the 3' wing segment and wherein each nucleotide (or nucleoside) of each wing segment comprises a modified sugar, such as a cEt bicyclic sugar.

It should be understood that any of the above modifications that can improve the properties of the antisense nucleic acid can be combined together.

For example, in a preferred embodiment, the nucleic acid molecule interfering specifically with dynamin 2 expression is an antisense nucleic acid that is 16 to 50 nucleobases in length and comprising any one of the sequences SEQ ID NO: 2873, SEQ ID NO: 3050, SEQ ID NO: 2117, SEQ ID NO: 2183, SEQ ID NO: 2447, SEQ ID NO: 2154 or SEQ ID NO: 2226, wherein the antisense further comprises:

a gap segment consisting of 10 linked 2'-deoxynucleotides (or 2'-deoxynucleosides);
a 5' wing segment consisting of 3 linked nucleotides (or nucleosides); and
a 3' wing segment consisting of 3 linked nucleotides (or nucleosides);

wherein the gap segment is positioned between the 5' wing segment and the 3' wing segment, wherein each nucleotide (or nucleoside) of each wing segment comprises a cEt sugar, wherein each internucleotide (or internucleoside) linkage is a phosphorothioate linkage, and wherein each cytosine is a 5-methylcytosine.

In a more preferred embodiment, the nucleic acid molecule interfering specifically with dynamin 2 expression is an antisense nucleic acid comprising or consisting of any one of the following sequences: Gks Tks Tks Tds Ads Tds Tds Ads Tds Ads Gds Gds Gds mCks Tks Tk (SEQ ID NO: 3129); Gks Gks Aks Tds Tds Tds Tds Ads Gds Gds Ads Gds Gds Tks Gks Ak (SEQ ID NO: 3130); Gks mCks Aks Tds Ads Gds Ads mCds Ads Ads Ads Tds mCds mCks mCks Ak (SEQ ID NO: 3131); Gks mCks Aks Ads Ads Tds Ads Tds Gds Ads Tds Tds mCds Aks Tks mCk (SEQ ID NO: 3132);

Gks Gks Tks mCds Ads Tds Tds Ads Ads Ads Gds Ads Tds Tks mCks Tk (SEQ ID NO: 3133); Aks Tks Gks Tds Ads Tds Tds Ads mCds mCds Tds Ads mCds Gks Gks mCk (SEQ ID NO: 3134); or Gks Tks Aks mCds Ads Ads Tds Gds Tds Ads Ads Gds mCds mCks Tks Tk (SEQ ID NO: 3135); wherein A is an adenine, mC is a 5-methylcytosine, G is a guanine, T is a thymine, k is a cEt sugar moiety, d is a 2'-deoxyribosyl sugar moiety, and s is a phosphorothioate internucleotide (or internucleoside) linkage.

In an even more preferred embodiment, the nucleic acid molecule interfering specifically with dynamin 2 expression is an antisense nucleic acid comprising or consisting of any one of the following sequences: Gks Tks Tks Tds Ads Tds Tds Ads Tds Ads Gds Gds Gds mCks Tks Tk (SEQ ID NO: 3129); Gks mCks Aks Tds Ads Gds Ads mCds Ads Ads Ads Tds mCds mCks mCks Ak (SEQ ID NO: 3131); wherein A is an adenine, mC is a 5-methylcytosine, G is a guanine, T is a thymine, k is a cEt sugar moiety, d is a 2'-deoxyribosyl sugar moiety, and s is a phosphorothioate internucleotide (or internucleoside) linkage.

In the most preferred embodiment, the nucleic acid molecule interfering specifically with dynamin 2 expression is an antisense nucleic acid comprising or consisting of the sequence Gks Tks Tks Tds Ads Tds Tds Ads Tds Ads Gds Gds Gds mCks Tks Tk (SEQ ID NO: 3129); wherein A is an adenine, mC is a 5-methylcytosine, G is a guanine, T is a thymine, k is a cEt sugar moiety, d is a 2'-deoxyribosyl sugar moiety, and s is a phosphorothioate internucleotide (or internucleoside) linkage.

Alternatively, an antisense nucleic acid can be designed to block a splice acceptor (SA) site and/or an exon splicing enhancer (ESE) and/or a branch point in a pre-mRNA and/or any sequence which could modulate pre-mRNA splicing, i.e. it can be designed to be complementary to a part of the pre-mRNA comprising an SA, an ESE, a branch point sequence or any sequence which could modulate pre-mRNA splicing.

For example, an antisense nucleic acid can be designed to induce exon-skipping within a pre-mRNA, thereby leading to a frameshift which produces a truncated cDNA containing a premature stop codon in the resulting mRNA. This strategy can be applied herein to the *DNM2* pre-mRNA, so as to reduce the level of DNM2 protein.

Accordingly, in a preferred embodiment, the nucleic acid molecule interfering specifically with dynamin 2 expression is an antisense nucleic acid that induces exon-skipping within a dynamin 2 pre-mRNA, such as exon 2 or exon 8 skipping. Skipping of exon 2 or exon 8 was indeed shown to lead to an absence of the dynamin 2 protein (Cowling et al., J Clin Invest. 2014;124(3): 1350-63; Tinelli et al., Hum Mol Genet. 2013; 22(21):4417-29).

Non-limiting examples of antisense nucleic acids inducing exon-skipping within a dynamin 2 pre-mRNA include, without limitation, those comprising or consisting of the sequence SEQ ID NO: 3136 (which induces DNM2 exon 2 skipping), or SEQ ID NO: 3137 (which induces DNM2 exon 8 skipping). To this end, said sequences may preferably be introduced within a U7 small nuclear RNA (U7 snRNA).

Other examples of antisense nucleic acids interfering specifically with dynamin 2 expression are those described in WO2018/189208, incorporated herein by reference in its entirety.

In another embodiment, RNAi can be used to down-regulate the expression of dynamin 2.

"*RNAi nucleic acid*" refers to a nucleic acid that can inhibit expression of a target gene by RNA interference (RNAi) mechanism. By contrast to antisense nucleic acids, RNAi nucleic acids target mature messenger RNA (mRNA). RNAi nucleic acids are well-known in the art, and include short-hairpin RNA (shRNA), small interfering RNA (siRNA), double-stranded RNA (dsRNA), and single-stranded RNA (ssRNA) (Sohail et al. 2004, Gene Silencing by RNA Interference: Technology and Application 1st Edition, ISBN 9780849321412; WO 99/32619; Wang et al., Pharm Res 2011, 28:2983-2995). RNA interference designates a phenomenon by which dsRNA specifically suppresses expression of a target gene at post-transcriptional level. In normal conditions, RNA interference is initiated by double-stranded RNA molecules (dsRNA) of several thousands of base pair length. *In vivo,* dsRNA - such as shRNA- introduced into a cell is cleaved by Dicer into a mixture of short interfering RNA called siRNA; the latter will bind to another enzyme (RISC) that will catalyze the cleavage of both the siRNA and target mRNA (Bernstein et al. Nature. 2001;409(6818):363-6). In mammalian cells, the siRNAs that are naturally produced by Dicer are typically 21-23 bp in length, with a 19 or 20 nucleotides duplex sequence, two-nucleotide 3' overhangs and 5'-triphosphate extremities (Zamore et al. Cell. 2000,101(l):25-33; Elbashir et al. Genes Dev. 2001, 15(2): 188-200; Elbashir et al. EMBO J. 2001, 20(23):6877-88). siRNA or shRNA are usually designed against a region 19 to 50 nucleotides downstream the translation initiator codon, whereas 5'UTR (untranslated region) and 3'UTR are usually avoided. The selected siRNA or shRNA target sequence should be subjected to a BLAST search against EST database to ensure that the only desired gene is targeted. Various products are commercially available to aid in the preparation and use of synthetic siRNA or shRNA. The RNAi nucleic acid can be of at least about 10 to 40 nucleotides (or nucleosides) in length, preferably about 15 to 30 base nucleotides (or nucleosides) in length. siRNA or shRNA can comprise naturally occurring RNA, synthetic RNA, or recombinantly produced RNA, as well as altered RNA that differs from naturally-occurring RNA by the addition, deletion, substitution and/or alteration of one or more nucleotides (or nucleosides). Such alterations can include addition of non-nucleotide (or non-nucleoside) material, such as to the end of the molecule or to one or more internal nucleotides (or nucleoside) of the siRNA, including modifications that make the siRNA resistant to nuclease digestion, as described above. Particularly preferred modified RNAi nucleic acids are those comprising at least one modified sugar, in particular in position 2' of

the nucleotide, such as 2'-O-methyl (2'-O-Me) or 2'fluoro (2'-F), as described in more details below.

Some dynamin 2 RNAi nucleic acids are commercially available. One can cite, for example, those commercialized by Abnova-Novus Biologicals (dynamin 2 RNAi No. H00001785-R05-H00001785-R08), or by Santa Cruz Biotechnology (dynamin II siRNA (h) No. sc-35236; dynamin II (h)-PR No. sc-35236-PR; dynamin II shRNA plasmid (h) No. sc-35236-SH; dynamin II shRNA (h) Lentiviral Particles No. sc-35236-V).

Thus, in a preferred embodiment, the nucleic acid molecule interfering specifically with dynamin 2 expression is an RNAi nucleic acid that is complementary to at least one part of DNM2 mRNA, in particular to at least one exon. The RNAi nucleic acid can be a siRNA or shRNA of at least about 10 to 40 nucleotides (or nucleosides) in length, preferably of about 15 to 30 nucleotides (or nucleosides) in length.

More preferably, the nucleic acid molecule interfering specifically with dynamin 2 expression is an RNAi nucleic acid that is complementary to at least exon 1, 4, 5, 12b, 13, 15, 17 and/or 21 of dynamin2 mRNA.

Even more preferably, the nucleic acid molecule interfering specifically with dynamin 2 expression is an RNAi nucleic acid comprising or consisting of a sequence selected from any one of SEQ ID NO: 3138 to SEQ ID NO: 3151.

Other examples of RNAi nucleic acids interfering specifically with Dynamin 2 expression are allele-specific siRNA described in WO2018/100010, incorporated herein by reference in its entirety.

In another embodiment, the nucleic acid interfering specifically with dynamin 2 expression is a ribozyme.

*"Ribozymes"* are catalytic RNA molecules with ribonuclease activity which are capable of cleaving a single-stranded nucleic acid, such as an mRNA, to which they have a complementary region. Thus, ribozymes can be used to catalytically cleave mRNA transcripts, thereby inhibiting translation of the protein encoded by the mRNA. Ribozyme molecules specific for a target (herein dynamin 2) can be designed, produced, and administered by methods commonly known to the art (see e.g., Fanning and Symonds (2006) RNA Towards Medicine (Handbook of Experimental Pharmacology), ed. Springer p. 289-303).

Yet, in another embodiment, genome editing can be used to inhibit dynamin 2. More specifically, one can use a genome editing system comprising a nuclease engineered to target the *Dnm2* gene.

*"Genome editing"* is a type of genetic engineering in which DNA is inserted, replaced, or removed from a genome using artificially engineered nucleases, also called molecular scissors. Nucleases create specific double-stranded break (DSBs) at desired locations in the genome, and harness the cell's endogenous mechanisms to repair the induced break by natural processes of homologous recombination (HR) or non-homologous end-joining (NHEJ). There are currently four known families of nucleases suitable for genome editing: zinc finger nucleases (ZFNs), transcription activator-like effector nucleases (TALENs), the CRISPR/Cas system in particular the Cas9 system (Mali et al, Nature Methods, 2013;10(10):957-63), or engineered meganucleases re-engineered homing endonucleases. Said nucleases can be delivered to the cells either as DNAs or mRNAs, such DNAs or mRNAs and can be engineered to target the *Dnm2* gene.

A particularly preferred genome editing system according to the invention is the CRISPR/Cas system in particular the Cas9 system.

In another embodiment, the dynamin 2 inhibitor used in the present invention is an antibody directed against dynamin 2.

An *"antibody"* is a polypeptide capable of specifically recognizing an antigen (herein, dynamin 2). To do so, the antibody's paratope interacts with the antigen's epitope. An antibody typically consists of four polypeptides - two full-length light chains and two full-length heavy chains - which are joined to one another with disulfide bonds to form a Y-shaped protein. Herein, the term antibody encompasses as well antibody fragments (Fab, Fv, ScFv, HCAb, sdAb, etc.) and antibody mimetics (ABDs, adhirons, affibodies, affimers, armadillo repeat proteins, DARPins, pronectins, transbodies, trimers X, etc.). This term also encompasses humanized and chimeric antibodies. Methods for preparing, using, and characterizing antibodies as defined herein are well known in the art (Immunobiology by Janeway et al., 5th edition, Garland publishing, 2001; Therapeutic Monoclonal Antibodies: From Bench to Clinic by An et al., Wiley editions, 2009; Skrlec et al. Trends in Biotechnology; 2015, 33(7): 408-41.; Harlow, E. and Lane, D. (1988) Antibodies: A Laboratory Manual, ed., Cold Spring Harbor Laboratory).

Examples of antibodies directed against dynamin 2 that are available, include, without limitation, antibodies sold or made by Novus Biologicals (catalogue No. NB300-617, NBP2-16244, (6C9) H00001785-M01), by Santa Cruz Biotechnology (catalogue No. sc-81150, sc-6400, sc-166525, sc-166669, sc-166526,) by BD-Biosciences (anti-DNM2 mouse ab, 610264), or by IGBMC-Illkirch (R2679, R2680, R2865, R2866, R2640, or R2641).

In another embodiment, the dynamin 2 inhibitor used in the present invention is a small molecule inhibiting the dynamin 2 enzymatic activity (i.e., inhibition of the GTPase activity), expression (such as by inhibiting promoter, splicing or translation) or function (such as inhibition of oligomerisation, activation, lipid binding, or partner binding).

As used throughout the specification, a *"small molecule"* refers to a low molecular weight compound, typically less than 1000 Daltons, which can be organic or inorganic. A small molecule can be derived from any known organism (including, but not limited to, animals, plants, bacteria, fungi and viruses) or from a library of synthetic molecules.

Small molecules inhibiting dynamin 2 activity, expression or function can be identified with the method described

herein. Small molecules that act as dynamin inhibitors are described in Harper et al. (Trends Cell Biol. 2013 Feb;23(2):90-101).

Examples of small molecules inhibiting dynamin 2 activity, expression or function that can be used according to the invention, include, without limitation, Dynasore (a noncompetitive, cell-permeable semicarbazone compound inhibitor of dynamin 1 and dynamin 2; CAS number 304448-55-3; chemical name: 3-hydroxynaphthalene-2-carboxylic acid (3,4-dihydroxybenzylidene)hydrazide); hydroxy-Dynasore (a highly potent inhibitor of dynamin 2 having a IC50 = 2.6 μM, which is a cell-permeable hydroxylated analog of Dynasore; CAS number 1256493-34-1; chemical name: 3-hydroxy-N'-[(2,4,5-trihydroxyphenyl) methylidene]naphthalene-2-carbohydrazide); tetradecyltrimethylammonium bromide (CAS number 1119-97-7; sold under the name MiTMAB™ (ab 120466) by Abcam; it is a cell permeable dynamin 1 and dynamin 2 inhibitor with an IC50 = 8.4 μM re. dynamin 2; it targets the pleckstrin homology domain and inhibits receptor-mediated and synaptic vesicle endocytosis); phthaladyn-23 (a cell-permeable phthalimide compound that inhibits dynamin 2 GTPase activity with an IC50 = 63 μM ; chemical name: 4-chloro-2-((2-(3-nitrophenyl)-1,3-dioxo-2,3-dihydro-1H-isoindole-5-carbonyl)-amino)-benzoic acid); Dynole 34-2 (a dynamin inhibitor V that acts on GTPase activity, noncompetitive for GTP; chemical name: 2-cyano-N-octyl-3-[1-(3-dimethylaminopropyl)- 1 H-indol-3 -yl] acrylamide), m-divi 1 (a mitochondrial division inhibitor with IC50 = 10μM ; chemical name: 3-(2,4-Dichloro-5-methoxyphenyl)-2-sulfanylquinazolin-4(3H)-one); iminodyn-22 (IC50 = 390nM; acts on a GTPase allosteric site and displays uncompetitive antagonism with respect to GTP; chemical name: N,N'-(Propane-1,3-diyl)bis(7,8-dihydroxy-2-imino-2H-chromene-3-carboxamide); iminodyn 17 (the chemical name: N,N'-(Ethane-1,2-diyl)bis(7,8-dihydroxy-2-imino-2H-chromene-3 -carboxamide); OcTMAB (it targets the PH domain of dynamin 2; chemical name: octadecylTriMethylAmmonium bromide); the dynamin inhibitory peptide with amino acid sequence QVPSRPNRAP (Tocris Biosciences 1774); Dyngo-4a (IC50 -2.5μM ; it acts on a GTPase allosteric site; chemical name :3-Hydroxy-N'-[(2,4,5-trihydroxyphenyl) methylidene]naphthalene-2-carbohydrazide); RTIL-13 (IC50 -2.3μM ; it is a norcantharidin scaffold targeting the PH domain of dynamin 2; chemical name: 4-(N,N-Dimethyl-N-octadecyl-N-ethyl)-4-aza-10-oxatricyclo-[5.2.1]decane-3,5-dione bromide).

The combination for use according to the invention comprises (i) any one of the dynamin 2 inhibitors as described above with (ii) any compound of formula (I) or an analog or a metabolite thereof, or a pharmaceutically acceptable salt, solvate, oxide, or stereoisomer thereof.

The compound of formula (I) is defined as follows:

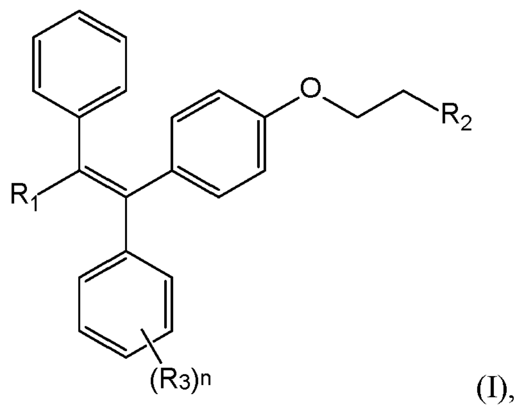

(I),

wherein

- $R_1$ is a radical selected from:
    - ◦ a $(C_1-C_6)$alkyl group optionally substituted by a halogen,
    - ◦ a hydrogen, or
    - ◦ a halogen;
- $R_2$ is a radical selected from:
    - ◦ a $-NR_4R_5$ group with $R_4$ and $R_5$ being independently a hydrogen, a $(C_1-C_6)$alkyl group, said $(C_1-C_6)$alkyl group being optionally substituted by a $-CF_3$ or a $-OCF_3$ group, or $R_4$ and $R_5$ form together a heterocycloalkyl, or
    - ◦ a $-OR_6$ group with $R_6$ being a H or a $(C_1-C_6)$alkyl group substituted by a hydroxy;
- n 0, 1, or 2; and/or
- $R_3$ is a radical selected from:

- a hydrogen,
- a hydroxy, or
- a halogen.

The term *"alkyl group"* refers to a saturated, linear or branched aliphatic group. The term *"($C_1$-$C_3$)alkyl"* more specifically means methyl, ethyl, propyl, or isopropyl. The term *"($C_1$-$C_6$)alkyl"* more specifically means methyl, ethyl, propyl, isopropyl, butyl, isobutyl, tert-butyl, pentyl or hexyl.

The term *"cycloalkyl"* corresponds to a saturated or unsaturated mono-, bi- or tri-cyclic alkyl group comprising between 3 and 20 atoms of carbons. It also includes fused, bridged, or spiro-connected cycloalkyl groups. The term *"cycloalkyl"* includes for instance cyclopropyl, cyclobutyl, cyclopentyl, and cyclohexyl. The term *"cycloalkyl"* may also refer to a 5-10 membered bridged carbocyclyl such as bicyclo[2,2,1]heptanyl, bicyclo[2,2,2]octanyl, bicyclo[1.1.1]pentanyl, or adamantly.

The term *"heterocycloalkyl"* corresponds to a saturated or unsaturated cycloalkyl group as defined above further comprising at least one heteroatom such as nitrogen, oxygen, or sulphur atom. It also includes fused, bridged, or spiro-connected heterocycloalkyl groups. Representative heterocycloalkyl groups include, but are not limited to 3-dioxolane, benzo [1,3] dioxolyl, azetidinyl, oxetanyl, pyrazolinyl, pyranyl, thiomorpholinyl, pyrazolidinyl, piperidyl, piperazinyl, 1,4-dioxanyl, imidazolinyl, pyrrolinyl, pyrrolidinyl, piperidinyl, imidazolidinyl, morpholinyl, 1,4-dithianyl, pyrrolidinyl, oxozolinyl, oxazolidinyl, isoxazolinyl, isoxazolidinyl, thiazolinyl, thiazolidinyl, isothiazolinyl, isothiazolidinyl, dihydropyranyl, tetrahydropyranyl, tetrahydrofuranyl, and tetrahydrothiophenyl.

By *"halogen",* it means herein a fluorine (F), chlorine (Cl), bromine (Br) or iodine (I) atom.

The expression *"substituted by"* means that the radical is substituted by one or several groups of the list.

As indicated above, analogs and metabolites of the compounds of formula (I) are encompassed by the present invention.

By *"analog",* it is meant herein a compound that is substantially similar to a native compound or compound of reference, for example in terms of physical, chemical, biochemical, and/or pharmacological properties, yet is not structurally identical to said native compound or compound of reference. Besides, analogs of a native compound or compound of reference do not necessarily have a similar structure to said native compound or compound of reference. In the context of the present invention, it shall be understood that the analog is active (i.e. functional), i.e. it retains all or part of the native activity of the native compound or compound of reference; herein, this means that the metabolite retains all or part of the activity (or lack thereof) of the tamoxifen with regard to at least lifespan, body weight, muscle strength, and/or DSS (Disease Severity Score). Such activity can be assessed by conventional methods in the art, such as according to the protocols described in the Materials and Methods section of Example 1 below.

The term *"metabolite"* refers to a an intermediate or end product of metabolism. More specifically, this term refers to a derivative of a native compound that is formed when said native compound is metabolized by a subject. In the context of the present invention, it shall be understood that the metabolite is active (i.e. functional), i.e. it retains all or part of the native activity of the native compound that has been metabolized; herein, this means that the metabolite retains all or part of the activity (or lack thereof) of the tamoxifen with regard to at least lifespan, body weight, muscle strength, and/or DSS (Disease Severity Score). Such activity can be assessed by conventional methods in the art, such as according to the protocols described in the Materials and Methods section of Example 1 below.

Pharmaceutically acceptable salts, solvates, oxides, and stereoisomers of the compounds of formula (I), analogs or metabolites thereof are also encompassed by the present invention.

The term *"pharmaceutically acceptable salt"* refers to a salt that is physiologically tolerated (i.e. non-toxic) when used in an appropriate manner in the context of the present invention. Pharmaceutically acceptable salts include those derived from pharmaceutically acceptable inorganic and organic acids and bases. Examples of suitable acids according to the invention include hydrochloric, hydrobromic, sulfuric, nitric, perchloric, fumaric, maleic, phosphoric, glycolic, lactic, salicylic, succinic, toluene-p-sulfuric, tartaric, acetic, citric, methane sulfonic, formic, benzoic, malonic, naphthalene-2-sulfuric and benzenesulfonic acids. Other acids, such as oxalic acid, while not themselves pharmaceutically acceptable, may be employed in the preparation of salts useful as intermediates in obtaining the compounds and their pharmaceutically acceptable acid addition salts. Salts derived from appropriate bases include alkali metal (e.g., sodium), alkaline earth metal, (e.g., magnesium), ammonium and N-($C_1$-$C_4$ alkyl)$_4^+$ salts.

Particularly preferred pharmaceutically acceptable salts according to the invention include, without limitation, citrate, tartrate and hydrochloride.

The term *"solvate "* according to the invention should be understood as meaning any form of the active compound (herein the compound of formula (I) or analog thereof), in which said compound is linked through non-covalent interactions to another molecule (normally a polar solvent), including especially hydrates and alcoholates, like for example, methanolate. Methods of solvation are well-known in the art.

An *"oxide "* refers herein to a compound in which at least one atom is oxidized such that said atom bears a positive formal charge and the attached oxygen atom bears a negative formal charge. For example, an *"N-oxide "* refers

to an oxide wherein the basic nitrogen atom of either a heteroaromatic ring or tertiary amine is oxidized to give a quaternary nitrogen bearing a positive formal charge and an attached oxygen atom bearing a negative formal charge.

A particularly preferred oxide according to the invention is an N-oxide.

*"Stereoisomers"* are chemical compounds that have the same molecular formula and sequence of bonded atoms, but differ in the 3D-dimensional orientations of their atoms in space. Stereoisomers include enantiomers (*R,* S configuration), as well as diastereoisomers such as *Cis-trans* and *E-Z* isomers, conformers, and anomers.

The designations "*R*" and "*S*" are commonly used in organic chemistry to denote specific configuration of a chiral center. The designations "*R*" refers to "right" and refers to that configuration of a chiral center with a clockwise relationship of group priorities (highest to second lowest) when viewed along the bond toward the lowest priority group. The term "*S*" or "left" refers to that configuration of a chiral center with a counterclockwise relationship of group priorities (highest to second lowest) when viewed along the bond toward the lowest priority group. The priority of groups for the R and S designation is based upon atomic number (heaviest isotope first). A partial list of priorities and a discussion of stereochemistry is contained in the book: The Vocabulary of Organic Chemistry, Orchin, et al. John Wiley and Sons, Inc., page 126 (1980), which is incorporated herein by reference in its entirety. It shall be further understood that the term enantiomer includes mixtures of *R* and *S* enantiomers (referred as *R*/*S*).

The designations "E" and "Z" are commonly used in organic chemistry to denote specific configuration of a double bond, formerly known as *"trans"* and "*cis*", respectively. According to the Cahn-Ingold-Prelog priority rules (CIP rules), each substituent on a double bond is assigned a priority, then positions of the higher of the two substituents on each carbon are compared to each other: if the two groups of higher priority are on opposite sides of the double bond (trans to each other), the bond is assigned the configuration E; by contrast, if the two groups of higher priority are on the same side of the double bond (cis to each other), the bond is assigned the configuration Z. It shall be further understood that the term E-Z isomer includes mixtures of E and Z isomers (referred as *E*/*Z*).

In a particular embodiment, the compound (ii) of formula (I) is such that $R_1$ is a radical selected from:

- a ($C_1$-$C_6$)alkyl group, preferably an ethyl group, optionally substituted by a halogen, preferably a chlorine; or
- a halogen, preferably a chlorine.

In a further particular embodiment, the compound (ii) of formula (I) is such that $R_2$ is a radical selected from:

- a -$NR_4R_5$ group with $R_4$ and $R_5$ being independently a hydrogen, a ($C_1$-$C_6$)alkyl group, preferably a methyl or an ethyl group, or $R_4$ and $R_5$ form together a pyrrolidinyl; or
- a -$OR_6$ group with $R_6$ being a H or a ($C_1$-$C_6$)alkyl group, preferably an ethyl group, substituted by a hydroxy.

In a further particular embodiment, the compound (ii) of formula (I) is such that n is 0. In a further particular embodiment, the compound (ii) of formula (I) is such that n is 1.

In a further particular embodiment, the compound (ii) of formula (I) is such that $R_3$ is a radical selected from: a hydrogen, a hydroxy, or a halogen, preferably a iodine.

Particularly preferred compounds (ii) of formula (I) according to the invention include, without limitation, those wherein:

- $R_1$ is an ethyl group, $R_2$ is a $NR_4R_5$ group with $R_4$ and $R_5$ being independently a methyl group, and n is 0 ;
- $R_1$ is an ethyl group substituted by a chlorine, $R_2$ is a $NR_4R_5$ group with $R_4$ and $R_5$ being independently a methyl group, and n is 0 ;
- $R_1$ is an ethyl group substituted by a chlorine, $R_2$ is a hydroxy, and n is 0;
- $R_1$ is an ethyl group, $R_2$ is a $NR_4R_5$ group with $R_4$ and $R_5$ being independently a methyl group, n is 1 and $R_3$ is a hydroxy;
- $R_1$ is an ethyl group, $R_2$ is a $NR_4R_5$ group with $R_4$ and $R_5$ forming together a pyrrolidinyl, n is 1 and $R_3$ is a iodine;
- $R_1$ is an ethyl group substituted by a chlorine, $R_2$ is a -$OR_6$ group with $R_6$ being an ethyl group substituted by a hydroxy, and n is 0 ; and
- $R_1$ is a chlorine, $R_2$ is a $NR_4R_5$ group with $R_4$ and $R_5$ being independently an ethyl, and n is 0.

Pharmaceutically acceptable salts, solvates, oxides, stereoisomers, analogs and metabolites thereof are also encompassed herein.

Particularly preferred analogs according to the invention include, without limitation:

- 6-(2,4-dichlorophenyl)-5-[4-[(3S)-1-(3-fluoropropyl)pyrrolidin-3-yl]oxyphenyl]-8,9-dihydro-7H-benzo[7]annulene-2-carboxylic acid;
- (5*R*,6*S*)-6-phenyl-5-[4-(2-pyrrolidin-1-ylethoxy)phenyl]-5,6,7,8-tetrahydronaphthalen-2-ol;
- [6-hydroxy-2-(4-hydroxyphenyl)-1-benzothiophen-3-yl]-[4-(2-piperidin-1-ylethoxy)phenyl]methanone;

- 1-[[4-[2-(azepan-1-yl)ethoxy]phenyl]methyl]-2-(4-hydroxyphenyl)-3-methylindol-5-ol;
- (*E*)-4-[2-[4-[(*E*)-1-(1H-indazol-5-yl)-2-phenylbut-1enyl]phenoxy]ethylamino]-N,N-dimethylbut-2-enamide; and
- (*E*)-3-[4-[(*E*)-2-(2-chloro-4-fluorophenyl)-1-(1H-indazol-5-yl)but-1-enyl]phenyl]prop-2-enoic acid.

Pharmaceutically acceptable salts, solvates, oxides, stereoisomers and metabolites thereof are also encompassed herein.

Particularly preferred metabolites according to the invention include, without limitation, compounds of formula (I) as defined above, wherein:

- $R_1$ is an ethyl group, $R_2$ is a $NR_4R_5$ group with $R_4$ and $R_5$ being independently a hydrogen and a methyl group, and n is 0;
- $R_1$ is an ethyl group, $R_2$ is a $NR_4R_5$ group with $R_4$ and $R_5$ being independently a hydrogen, and n is 0;
- $R_1$ is an ethyl group, $R_2$ is a $NR_4R_5$ group with $R_4$ and $R_5$ being independently an ethyl group, n is 1 and $R_3$ is a hydroxy;
- $R_1$ is an ethyl group, $R_2$ is a $NR_4R_5$ group with $R_4$ and $R_5$ being independently a hydrogen and a methyl group, n is 1 and $R_3$ is a hydroxy; and
- $R_1$ is an ethyl group, $R_2$ is a $NR_4R_5$ group with $R_4$ and $R_5$ being independently a hydrogen, n is 1 and $R_3$ is a hydroxy;

as well as 4-[1-(4-hydroxyphenyl)-2-phenylbut-1-en-1-yl]phenol and 4-(1,2-diphenylbut-1-enyl)phenol.

Pharmaceutically acceptable salts, solvates, oxides, and stereoisomers thereof are also encompassed herein.

In a preferred embodiment, the compound (ii) of the invention is a compound of formula (I) as defined above, wherein $R_1$ is an ethyl group, $R_2$ is a $NR_4R_5$ group with $R_4$ and $R_5$ being independently a methyl group, and n is 0 ; or is an analog or a metabolite thereof; or is a pharmaceutically acceptable salt, solvate, oxide, or stereoisomer thereof.

In a more preferred embodiment, the compound (ii) of the invention is selected from the group consisting of:

- 2-[4-[(*Z*)-1,2-diphenylbut-1-enyl]phenoxy]-N,N-dimethylethanamine;
- 6-(2,4-dichlorophenyl)-5-[4-[(3*S*)-1-(3-fluoropropyl)pyrrolidine-3-yl]oxyphenyl]-8,9-dihydro-7H-benzo[7]annulene-2-carboxylic acid;
- (5*R*,6*S*)-6-phenyl-5-[4-(2-pyrrolidin-1-ylethoxy)phenyl]-5,6,7,8-tetrahydronaphthalen-2-ol;
- [6-hydroxy-2-(4-hydroxyphenyl)-1-benzothiophen-3-yl]-[4-(2-piperidin-1-ylethoxy)phenyl]methanone;
- 1-[[4-[2-(azepan-1-yl)ethoxy]phenyl]methyl]-2-(4-hydroxyphenyl)-3-methylindol-5-ol;
- (*E*)-4-[2-[4-[(*E*)-1-(1H-indazol-5-yl)-2-phenylbut-1enyl]phenoxy]ethylamino]-N,N-dimethylbut-2-enamide; and
- (*E*)-3-[4-[(*E*)-2-(2-chloro-4-fluorophenyl)-1-(1H-indazol-5-yl)but-1-enyl]phenyl]prop-2-enoic acid.

Pharmaceutically acceptable salts, solvates, oxides, stereoisomers and metabolites thereof are also encompassed herein.

Yet, in an even more preferred embodiment, the compound (ii) of formula (I) is 2-[4-[(Z)-1,2-diphenylbut-1-enyl]phenoxy]-N,N-dimethylethanamine, or a pharmaceutically acceptable salt thereof.

The following Table 1 illustrates, in a non-limitative way, some chemical structures of the compounds (ii) according to the invention. Said compounds can be either commercially purchased or prepared according to methods well-known in the art (e.g. UK1064629; Bedford et al., Nature, 1996: 212; EP0168175A1; Tandon et al., Asian Journal or Organic Chemistry; 2020, doi: 10.1002/ajoc.202000308).

**Table 1. Compounds (ii) according to the invention**

| Compound (ii) | Structure | CAS number |
|---|---|---|
| tamoxifen (also known as 2-[4-[(*Z*)-1,2-diphenylbut-1-enyl]phenoxy]-N,N-dimethylethanamine) | 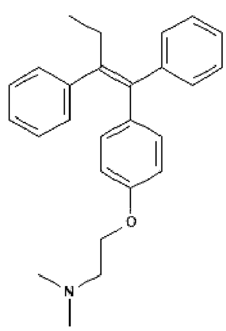 | 10540-29-1 |

(continued)

| Compound (ii) | Structure | CAS number |
|---|---|---|
| tamoxifen citrate (also known as 2-[4-[(*Z*)-1,2-diphenylbut-1-enyl]phenoxy]-N,N-dimethylethanamine; 2-hydroxypropane-1,2,3-tricarboxylic acid) | 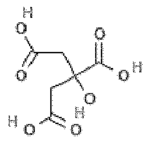 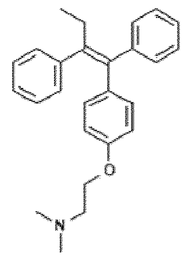 | 54965-24-1 |
| 4-chlorotamoxifen (also known as toremifene or 2-[4-[(*Z*)-4-chloro-1,2-diphenylbut-1-enyl]phenoxy]-N,N-dimethylethanamine) | 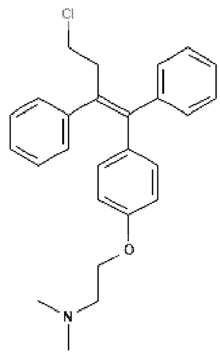 | 89778-26-7 |
| 4-chlorotamoxifen citrate (also known as toremifene citrate or 2-[4-[(*Z*)-4-chloro-1,2-diphenylbut-1-enyl] phenoxy]-N,N-dimethylethanamine; 2-hydroxypropane-1,2,3-tricarboxylic acid) | 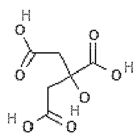 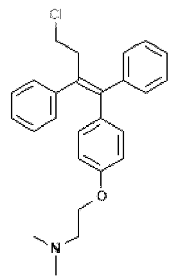 | 89778-27-8 |
| ospemifene (also known as 2-[4-[(*Z*)-4-chloro-1,2-diphenylbut-1-enyl]phenoxy]ethanol) | 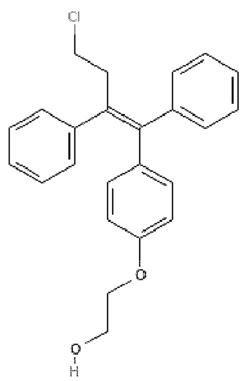 | 128607-22-7 |

(continued)

| Compound (ii) | Structure | CAS number |
|---|---|---|
| droloxifene (also known as 3-[(*E*)-1-[4-[2-(dimethylamino)ethoxy]phenyl] -2-phenylbut-1-enyl]phenol) | 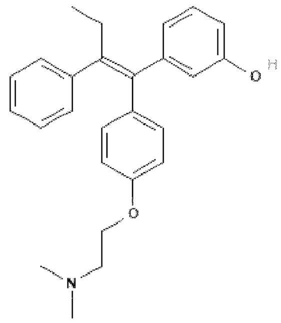 | 82413-20-5 |
| idoxifene (also known as 1-[2-[4-[(*E*)-1-(4-iodophenyl)-2-phenylbut-1-enyl]phenoxy]ethyl]pyrrolidine) | 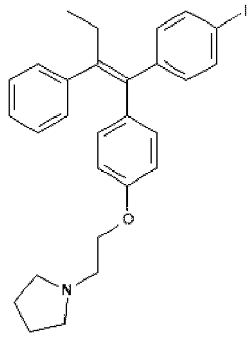 | 116057-75-1 |
| fispemifene (also known as 2-[2-[4-[(*Z*)-4-chloro-1,2-diphenylbut-1-enyl]phenoxy]ethoxy]ethanol) | 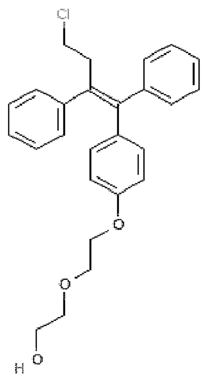 | 341524-89-8 |
| clomiphene (also known as 2-[4-[(*E*)-2-chloro-1,2-diphenylethenyl]phenoxy] -N,N-diethylethanamine) | 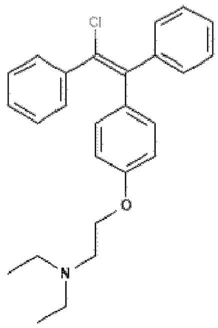 | 15690-57-0 |

(continued)

| Compound (ii) | Structure | CAS number |
|---|---|---|
| N-desmethyltamoxifen (also known as 2-[4-[(*Z*)-1,2-diphenylbut-1-enyl]phenoxy]-N-methylethanamine) | 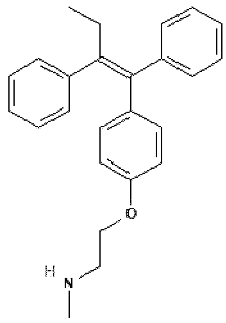 | 31750-48-8 |
| desdimethyltamoxifen (also known as 2-[4-[(*Z*)-1,2-diphenyl but-1-enyl] phenoxy]ethanamine) | 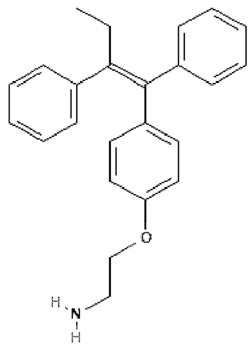 | 80234-20-4 |
| 4-hydroxytamoxifen (also known as afimoxifene or 4-[(*Z*)-1-[4-[2-(dimethylamino)ethoxy]phenyl]-2-phenylbut-1-enyl]phenol) | 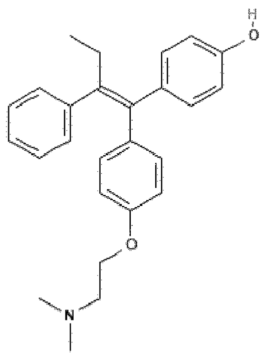 | 68047-06-3 |
| endoxifen (also known as 4-[(*Z*)-1-[4-[2-(methylamino) ethoxy]phenyl]-2-phenylbut-1-enyl]phenol) | 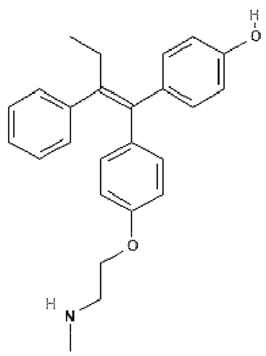 | 112093-28-4 |

(continued)

| Compound (ii) | Structure | CAS number |
|---|---|---|
| norendoxifen (also known as 4-[(*Z*)-1-[4-(2-aminoethoxy) phenyl]-2-phenylbut-1-enyl] phenol) | 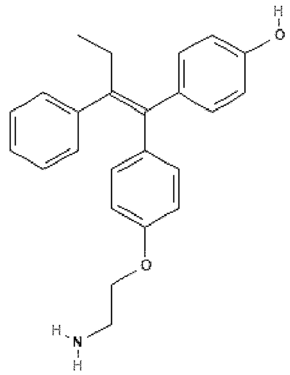 | 1308808-22-1 |
| tamoxifene bisphenol (also known as 4-[1-(4-hydroxyphenyl)-2-phenylbut-1-en-1-yl]phenol) | 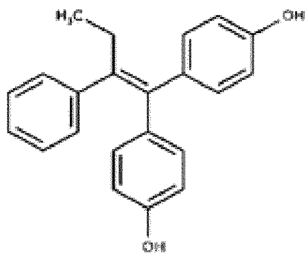 | 4120-45-0 |
| metabolite E (also known as 4-(1,2-diphenylbut-1-enyl) phenol) | 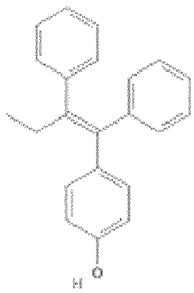 | 68684-63-9 |
| amcenestrant (also known as 6-(2,4-dichlorophenyl)-5-[4-[(3S)-1-(3-fluoropropyl)pyrrolidine-3-yl]oxyphenyl]-8,9-dihydro-7H-benzo[7]annulene-2-carboxylic acid) | 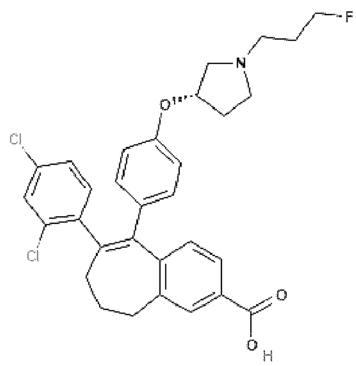 | 2114339-57-8 |

(continued)

| Compound (ii) | Structure | CAS number |
|---|---|---|
| lasofoxifene (also known as (5R,6S)-6-phenyl-5-[4-(2-pyrrolidin-1-ylethoxy)phenyl]-5,6,7,8-tetrahydronaphthalen-2-ol) | 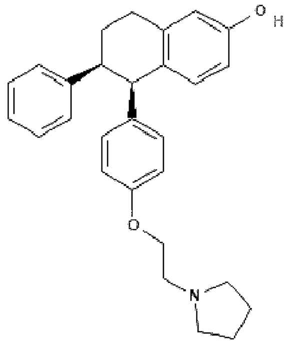 | 180916-16-9 |
| lasoxifene tartrate (also known as (2S,3S)-2,3-dihydroxybutanedioic acid;(5R,6S)-6-phenyl-5-[4-(2-pyrrolidin-1-ylethoxy)phenyl]-5,6,7,8-tetrahydro naphthalen-2-ol) | 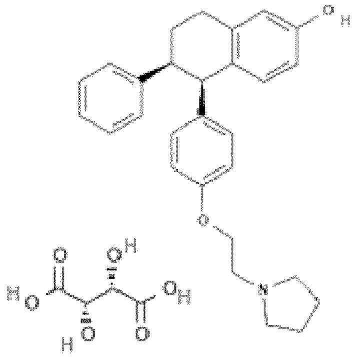 | 190791-29-8 |
| raloxifene (also known as [6-hydroxy-2-(4-hydroxyphenyl)-1-benzothiophen-3-yl]-[4-(2-piperidin-1-ylethoxy)phenyl]methanone) | 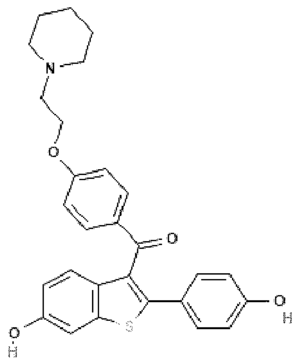 | 84449-90-1 |
| raloxifene hydrochloride (also known as [6-hydroxy-2-(4-hydroxyphenyl)-1-benzothiophen-3-yl]-[4-(2-piperidin-1-ylethoxyphenyl] methanone;hydrochloride) | 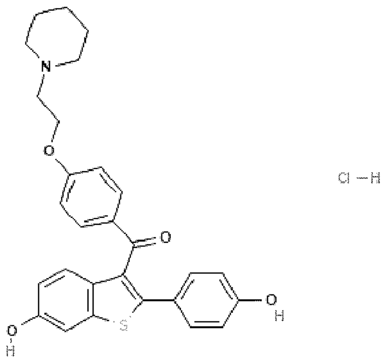 | 82640-04-8 |

(continued)

| Compound (ii) | Structure | CAS number |
|---|---|---|
| bazedoxifene (also known as 1-[[4-[2-(azepan-1-yl) ethoxy]phenyl]methyl]-2-(4-hydroxyphenyl)-3-methylindol-5-ol) | 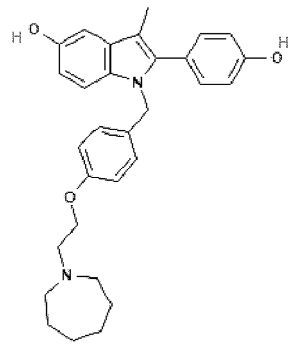 | 198481-32-2 |
| bazedoxifene hydrochloride (also known as 1-[[4-[2-(azepan-1-yl)ethoxy]phenyl]methyl]-2-(4-hydroxyphenyl)-3 -methylindol-5 - ol;hydrochloride) | 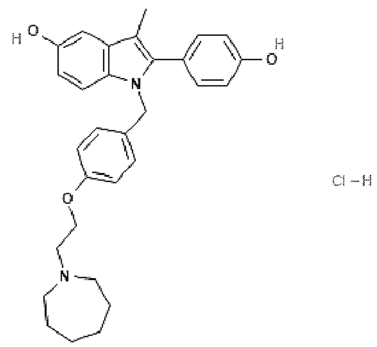 | 198480-56-7 |
| H3B-5942 (also known as (*E*)--4-[2-[4-[(E)-1-(1H-indazol-5-yl)-2-phenylbut-1-enyl]phenoxy] ethylamino]-N,N-dimethylbut-2-enamide) | 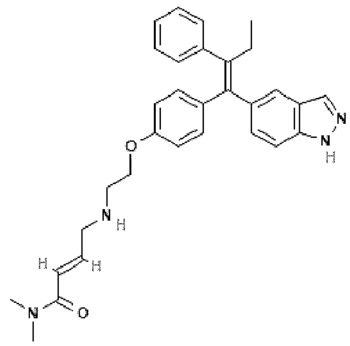 | 2052128-15-9 |
| brilanestrant (also known as (*E*)-3-[4-[(*E*)-2-(2-chloro-4-fluorophenyl)-1-(1H-indazol-5-yl)but-1-enyl]phenyl] prop-2-enoic acid) | 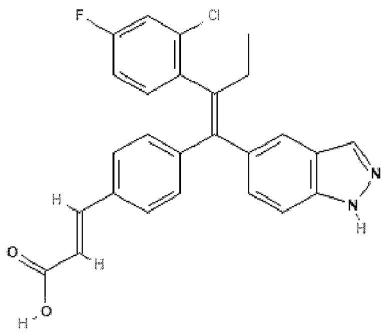 | 1365888-06-7 |

The skilled person would readily understand that any one of the (i) dynamin 2 inhibitors and (ii) compounds of formula (I) or analogs or metabolites thereof, or pharmaceutically acceptable salts, solvates, oxides, or stereoisomer thereof, as described herein, can be combined for the purpose of the present invention.

In a particularly preferred embodiment:

(i) the dynamin 2 inhibitor is a nucleic acid molecule interfering specifically with dynamin 2 expression; and
(ii) the compound is of formula (I) or is an analog or a metabolite thereof, or a pharmaceutically acceptable salt, solvate, oxide, or stereoisomer thereof.

In a further preferred embodiment:

(i) the nucleic acid molecule interfering specifically with dynamin 2 expression is an antisense nucleic acid; and
(ii) the compound is of formula (I) or an analog or a metabolite thereof,
wherein the compound of formula (I) is such that:

- $R_1$ is a radical selected from: a ($C_1$-$C_6$)alkyl group, preferably an ethyl group, optionally substituted by a halogen, preferably a chlorine; or a halogen, preferably a chlorine;
- $R_2$ is a radical selected from: a -$NR_4R_5$ group with $R_4$ and $R_5$ being independently a hydrogen, a ($C_1$-$C_6$)alkyl group, preferably a methyl or an ethyl group, or $R_4$ and Rs form together a pyrrolidinyl; or a -$OR_6$ group with $R_6$ being a H or a ($C_1$-$C_6$)alkyl group, preferably an ethyl group, substituted by a hydroxy;
- n is 0 or 1; and/or
- $R_3$ is a radical selected from: a hydrogen, a hydroxy, or a halogen, preferably a iodine;

or is a pharmaceutically acceptable salt, solvate, oxide, or stereoisomer thereof.

In a more preferred embodiment:

(i) the antisense nucleic acid interfering specifically with dynamin 2 expression is complementary to at least one intron or the 3'UTR of Dynamin 2 pre-mRNA, preferably of at least intron 12 of dynamin 2 pre-mRNA; and
(ii) the compound is of formula (I) or is an analog or a metabolite thereof,

wherein the compound of formula (I) is such that $R_1$ is an ethyl group, $R_2$ is a $NR_4R_5$ group with $R_4$ and $R_5$ being independently a methyl group, and n is 0;
or is a pharmaceutically acceptable salt, solvate, oxide, or stereoisomer thereof.

Yet, a further preferred embodiment:

(i) the antisense nucleic acid interfering specifically with dynamin 2 expression comprises or consisting of at least 8 contiguous nucleobases of any one of SEQ ID NO: 1 to 3135, and
(ii) the compound is selected from the group consisting of:

- 6-(2,4-dichlorophenyl)-5-[4-[(3*S*)-1-(3-fluoropropyl)pyrrolidin-3-yl]oxyphenyl]-8,9-dihydro-7H-benzo[7]annulene-2-carboxylic acid;
- (5*R*,6*S*)-6-phenyl-5-[4-(2-pyrrolidin-1-ylethoxy)phenyl]-5,6,7,8-tetrahydronaphthalen-2-ol;
- [6-hydroxy-2-(4-hydroxyphenyl)-1-benzothiophen-3-yl]-[4-(2-piperidin-1-ylethoxy)phenyl]methanone;
- 1-[[4-[2-(azepan-1-yl)ethoxy]phenyl]methyl]-2-(4-hydroxyphenyl)-3-methylindol-5-ol;
- (*E*)-4-[2-[4-[(*E*)-1-(1H-indazol-5-yl)-2-phenylbut-1enyl]phenoxy]ethylamino]-N,N-dimethylbut-2-enamide;
- (*E*)-3-[4-[(*E*)-2-(2-chloro-4-fluorophenyl)-1-(1H-indazol-5-yl)but-1-enyl]phenyl]prop-2-enoic acid; and
- pharmaceutically acceptable salts thereof.

In a further preferred embodiment:

(i) the antisense nucleic acid interfering specifically with dynamin 2 expression comprises the sequence SEQ ID NO: 2873 or SEQ ID NO: 3129, and
(ii) the compound is selected from the group consisting of:

- 6-(2,4-dichlorophenyl)-5-[4-[(3*S*)-1-(3-fluoropropyl)pyrrolidin-3-yl]oxyphenyl]-8,9-dihydro-7H-benzo[7]annulene-2-carboxylic acid;
- (5*R*,6*S*)-6-phenyl-5-[4-(2-pyrrolidin-1-ylethoxy)phenyl]-5,6,7,8-tetrahydronaphthalen-2-ol;
- [6-hydroxy-2-(4-hydroxyphenyl)-1-benzothiophen-3-yl]-[4-(2-piperidin-1-ylethoxy)phenyl]methanone;
- 1-[[4-[2-(azepan-1-yl)ethoxy]phenyl]methyl]-2-(4-hydroxyphenyl)-3-methylindol-5-ol;
- (*E*)-4-[2-[4-[(*E*)-1-(1H-indazol-5-yl)-2-phenylbut-1enyl]phenoxy]ethylamino]-N,N-dimethylbut-2-enamide;
- (*E*)-3-[4-[(*E*)-2-(2-chloro-4-fluorophenyl)-1-(1H-indazol-5-yl)but-1-enyl]phenyl]prop-2-enoic acid; and

- pharmaceutically acceptable salts thereof.

In a particularly preferred embodiment:

(i) the antisense nucleic acid interfering specifically with dynamin 2 expression comprises the sequence SEQ ID NO: 2873 or SEQ ID NO: 3129, and
(ii) the compound of formula (I) is 2-[4-[(*Z*)-1,2-diphenylbut-1-enyl]phenoxy]-N,N-dimethylethanamine, or a pharmaceutically acceptable salt thereof.

The combination comprising (i) a dynamin 2 inhibitor and (ii) a compound of formula (I) or an analog or a metabolite thereof, or a pharmaceutically acceptable salt, solvate, oxide, or stereoisomer thereof, as described herein, may be suitable for administration to a cell, tissue and/or an organ of individuals affected by or at risk of suffering from a centronuclear myopathy or a dystrophinopathy, preferably a centronuclear myopathy, and may be administered *in vivo, ex vivo* or *in vitro.* Since centronuclear myopathies and dystrophinopathies affect muscles, it is preferred that said cells are muscle cells, that said tissue is a muscular tissue and/or that said organ comprises or consists of a muscular tissue.

Whether the combination is administered simultaneously, separately or sequentially, each of therapy can be delivered as it is to the subject, or be formulated to be compatible with their intended route of administration.

For example, the combination can be formulated in a combined pharmaceutical composition, or in separate pharmaceutical compositions, in a form suitable for parenteral, oral or topical administration, such as a liquid suspension, a solid dosage form (granules, pills, capsules or tablets), or a paste or gel.

For the purposes of the invention, a particularly preferred form of administration is parenteral administration, such as subcutaneous, intradermal, intravenous, or intramuscular administration. Intramuscular administration, in particular by injection, is herein preferred.

It is within the skill of the person in the art to formulate such pharmaceutical composition(s) in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York). Pharmaceutical compositions according to the invention may notably be formulated to release the combination therapy immediately upon administration or at any predetermined time or time period after administration.

When formulated within a pharmaceutical composition, the combination can be further combined with a pharmaceutically acceptable excipient.

As used herein, the term a "*pharmaceutically acceptable excipient*" means an inactive or inert, and therefore nontoxic, component, as it has no pharmacological action itself, which can be used to improve properties of a composition, such as shelf-life, retention time at the application site, consumer acceptance, etc. It includes, without limitation, surfactants (cationic, anionic, or neutral); surface stabilizers; other enhancers, such as preservatives, wetting or emulsifying agents; solvents; buffers; salt solutions; dispersion medium; isotonic and absorption delaying agents, and the like; that are physiologically compatible.

As explained above, the combination is provided in a therapeutically effective amount so as to treat the centronuclear myopathy or dystrophinopathy.

In a preferred embodiment, the therapeutically effective amount of dynamin 2 inhibitor is an amount sufficient to reduce the dynamin 2 expression, activity or function in a level equal or less than the normal level. The normal level of reference is the level of dynamin 2 expression, activity or function of subjects that do not suffer from a centronuclear myopathy or dystrophinopathy.

In a preferred embodiment, the therapeutically effective amount of dynamin 2 inhibitor is a pediatric dose in monotherapy, or a dose that is subtherapeutic in monotherapy (yet therapeutically effective in the combination of the invention).

For example, the therapeutically effective amount of dynamin 2 inhibitor is such that it reduces the dynamin 2 expression, activity or function by 20% to 30% compared to the normal level.

In a preferred embodiment, the therapeutically effective amount of compound (ii) is an amount that does not reduce, or at least substantially reduce, the dynamin 2 expression, activity or function, such as to a level equal or less than the normal level (yet therapeutically effective in the combination of the invention).

In a preferred embodiment, the therapeutically effective amount of compound (ii) is a pediatric dose in monotherapy, or a dose that is subtherapeutic in monotherapy (yet therapeutically effective in the combination of the invention).

For example, a daily dose of about 1.5 mg/kg (body weight) or of about 20 mg of compound (ii) can be considered as a therapeutically effective dose in the combination according to the invention.

Those of skill in the art will recognize that such parameters are normally worked out during clinical trials.

In another aspect, the present invention relates to (i) a dynamin 2 inhibitor and (ii) a compound of formula (I)

or an analog or a metabolite thereof, or a pharmaceutically acceptable salt, solvate, oxide, or stereoisomer thereof, as described above, as a combined preparation for simultaneous, separate or sequential use in the treatment of a myopathy selected from a centronuclear myopathy or a dystrophinopathy, preferably a centronuclear myopathy.

The invention relates to the simultaneous, separate or sequential use of (i) a dynamin 2 inhibitor and (ii) a compound of formula (I) or an analog or a metabolite thereof, or a pharmaceutically acceptable salt, solvate, oxide, or stereoisomer thereof, as described above, for the preparation of a medicament for the treatment of a myopathy selected from a centronuclear myopathy or a dystrophinopathy, preferably a centronuclear myopathy.

Further provided is therefore a method for treating a myopathy selected from a centronuclear myopathy or a dystrophinopathy, preferably a centronuclear myopathy, in a subject in need thereof, said method comprising the simultaneous, separate or sequential administration, to said subject, of a therapeutically effective amount of (i) a dynamin 2 inhibitor and (ii) a compound of formula (I) or an analog or a metabolite thereof, or a pharmaceutically acceptable salt, solvate, oxide, or stereoisomer thereof, as described above.

Preferred embodiments, as described above, apply herein *mutatis mutandis.*

In another aspect, the present invention relates to a pharmaceutical composition comprising (i) a dynamin 2 inhibitor and (ii) a compound of formula (I) or an analog or a metabolite thereof, or a pharmaceutically acceptable salt, solvate, oxide, or stereoisomer, thereof, and optionally (iii) a pharmaceutically acceptable excipient.

Preferred embodiments, as described above, apply herein *mutatis mutandis.*

The present invention will be better understood in the light of the following detailed experiments. Nevertheless, the skilled artisan will appreciate that the present examples are not limitative and that various modifications, substitutions, omissions, and changes may be made without departing from the scope of the invention.

## EXAMPLES

### EXAMPLE 1

1. MATERIALS AND METHODS

#### *1.1. Antisense oligonucleotide and tamoxifen*

ASO targeting *Dnm2*

The antisense oligonucleotide targeting *Dnm2* (ASO-Dnm2) was selected for its capacity to efficiently knockdown DNM2, as previously validated by Tasfaout et al. (Nat Commun 2017, 8: 15661) and Buono et al. (Proc Natl Acad Sci USA 2018, 115(43): 11066-11071). This ASO more specifically targets exon 17 of the *Dnm2* mouse gene. A random control ASO sequence not targeting any known mouse gene was used as a control (ASO-ctrl). These oligonucleotides were synthesized using an automated DNA synthesizer, chemically modified with phosphorothioate in the backbone and cEt modifications on the wings with a deoxy gap (3-10-3 design) and purified, as previously described (Tasfaout, et al., Nat Commun, 2017; 8: 15661; Seth et al., Nucleic Acids Symp Ser (Oxf), 2008; 52: 553-554).

All ASOs used in the present study are described in Table 2 below. These ASOs were dissolved in filtered and autoclaved in sterile D-PBS (Life Technologies, #14190-144).

For experiments in mice, the ASOs were injected intraperitonally (i.p.) weekly from 4 weeks to 12 weeks of age at 3 mg/kg.

For experiments in mouse immortalized myoblasts (C2C12 cells), the ASOs were electroporated at 2 μM in 2 mm cuvettes, at 150V for 5 ms (one pulse) for 2 million cells in a volume of 400 μl PBS, or in 4mm cuvettes, at 300V for 5 ms (one pulse) for 4 million cells in a volume of 800 μl PBS.

**Table 2. Antisense oligonucleotides**

| ASO name | ASO target | ASO sequence (5' to 3') (SEQ ID NO:) | ASO chemistry |
|---|---|---|---|
| ASO-ctrl (control) | None | GGCCAATACGCCGTCA (SEQ ID NO: 3152) | Phosphorothiorate Constrained ethyl (cEt) |
| ASO-Dnm2 | *Dnm2* | GGCATAAGGTCACGGA (SEQ ID NO: 3153) | Phosphorotiorate Constrained ethyl (cEt) |

Tamoxifen

For experiments in mice, pellets of diet were supplemented with tamoxifen citrate (SAFE; 0.1g/kg tamoxifen) to achieve 10 mg of tamoxifen free base per kg of chow. Pellets of diet devoid of tamoxifen were used as a control (to be administered with ASO-ctrl).

For experiments conducted in mouse immortalized myoblasts (C2C12 cells), 4-hydroxytamoxifen (5 mg/ml solution, Sigma #SML-1666) was used. The stock solution at 13mM in ethanol:isopropanol (95:5) was diluted in cell culture medium to obtain a working concentration of 20 $\mu$M. For vehicle control cells (no treatment), ethanol:isopropanol (95:5) was added to the cell culture medium.

### *1.2. Animal experiments*

All procedures involving mice complied with French and European legislation on animal care and experimentation.

*Mtm1* deficient mice in the 129Pas genetic background were obtained from Dr. Laporte (IGBMC, Illkirch, France). Wild type males were bred with *Mtm1*$^{+/-}$ heterozygous females. The *Mtm1*$^{+/y}$ (wild type, i.e. WT) and *Mtm1*$^{-/y}$ (mutant) males in the progeny were used in the experiments. The *Mtm1*$^{-/y}$ knock-out mice reproduce the hallmarks of XL-CNM patients such as abnormal position of the organelles, defects in the localization of the nuclei and muscle atrophy, associated with progressive muscle weakness including respiratory weakness, leading to death between 6 and 14 weeks of age (Buj-Bello et al., Proc Natl Acad Sci USA, 2002; 99: 15060-15065; Al-Qusairi,et al, Proc Natl Acad Sci USA, 2009; 106: 18763-18768).

Animals were followed for up to 12 weeks old, with weekly recordings of clinical signs and survival. Treatments were started when disease started to manifest, i.e. at 4 weeks of age.

Weekly i.p. injections of 3 mg/kg of antisense were performed in the *Mtm1*$^{+/y}$ and *Mtm1*$^{-/y}$ mice, from 4 to 12 weeks of age. Alongside these injections, mice were fed pellets of diets containing tamoxifen citrate (10 mg/kg) or devoid of tamoxifen.

The 3 mg/kg ASO dose was chosen to achieve a knockdown of *Dnm2* RNA of about 15% to 20%, corresponding to the low clinical range; while the 10 mg/kg of diet for tamoxifen was chosen to correspond to the low pediatric clinical range (which typically corresponds to about 5-10 mg/day for a 60 kg human).

### *1.3. Lifespan*

Mice were followed for up to 12 weeks old, with recordings of their lifespan.

### *1.4. Body weight*

Mice were followed for up to 12 weeks old, with weekly recordings of their body weight.

### *1.5. Hanging test*

Whole-body strength of mice was evaluated using the hanging test. Briefly, each mouse was placed in the middle of a grid and the grid was turned upside down above a cage with bedding. The suspending animal should hold on to the grid with all four limbs in order to avoid falling. The grid was placed at a height of approximately 40 cm above the bedding to make sure that the mouse was not influenced to jump. The latency time for the mouse to fall in the cage was measured, with a cut-off time of 60 seconds. Each animal was given a maximum of 3 trials per session, with 5 minutes minimum recovery period between trials. The maximum hanging time was recorded.

The hanging test was performed before treatment, on the first day of treatment (4 weeks old), and 48 hours after treatment every week until 12 weeks old.

### *1.6. Disease severity Score (DSS)*

Disease Severity Score was calculated to monitor the clinical appearance of mice (n = 10 per group). The DSS was designed to evaluate the clinical evolution of four CNM features, based on a previously described protocol (Tasfaout, et al., Nat Commun, 2017; 8: 15661) that has been optimized (Buono et al., Proc Natl Acad Sci USA 2018, 115(43): 11066-11071) and further modified as follows (Buono et al., Dis Model Mech. 2022; doi: 10.1242/dmm.049284):

- breathing difficulties and ptosis were not included in the scoring;
- the hanging test ability score was calculated with the formula

$$[(60\text{-Time (s))}/60s]*2 = \text{value (0-2)} ;$$

- the body weight score was calculated as the change in body weight of *Mtm1*$^{-/y}$ mice from week n to week n+1 (named x) with score:
  0: x ≥ 0.25g, score 0.5: -0.25 > x > 0.25, score 1: x ≤ -0.25g;

- no change for kyphosis and walking ability.

The score varies from 0 (no disease) to 5 (most severe).

### *1.7. Cell culture experiments*

The C1C12 immortalized myoblast cells were cultured in DMEM with 1g/l glucose (Gibco) with 20% FBS (Gibco) + 40 µg/ml Gentamycin (Gibco). The cells were electroporated with either PBS, ASO-ctrl or ASO-Dnm2 at 2 µM, and/or treated with 4-OH-tamoxifen at 20 µM in the cell culture medium, as described in 1.1. The experiment was done in duplicate wells, in 6-well plates. After 24 hrs of treatment, cells were collected and either RNA was extracted for RT-qPCR or protein was extracted for Western Blot.

### *1.8. RT-qPCR*

RNA was isolated from myoblast C2C12 cells, treated with either (i) PBS, (ii) PBS + Tamoxifen (20 µM), (iii) ASO ctrl (2 µM), (iv) ASO ctrl (2 µM) + Tamoxifen (20 µM), (v) ASO-Dnm2 (2 µM) or (vi) ASO-Dnm2 (2 µM) + Tamoxifen (20 µM), using Nucleospin RNA Mini Kit (Macherey-Nagel)), according to the manufacturer's instructions. Reverse transcription was carried out on a 250 ng aliquot using SuperScript™ IV Reverse Transcriptase (Invitrogen).

RT-qPCR was performed in a Quant Studio 5 Real-Time PCR System (Thermo Scientific), using the primers (100 µM) of Table 3 mixed in PowerUp™ SYBR™ Green Master Mix (Applied Biosystems). The relative expression of *Dnm2* mRNA was normalized to *Rpl27* and *Tbp*.

**Table 3. Primers used for RT-qPCR**

| Gene | Forward primer (5' to 3') (SEQ ID NO:) | Reverse primer (5' to 3') (SEQ ID NO:) |
|---|---|---|
| *Rpl27* | AAGCCGTCATCGTGAAGAACA (SEQ ID NO: 3154) | CTTGATCTTGGATCGCTTGGC (SEQ ID NO: 3155) |
| *Tbp* | ACCGTGAATCTTGGCTGTAAAC (SEQ ID NO: 3156) | GCAGCAAATCGCTTGGGATTA (SEQ ID NO: 3157) |
| *Dnm2* | ACCCCACACTTGCAGAAAAC (SEQ ID NO: 3158) | CGCTTCTCAAAGTCCACTCC (SEQ ID NO: 3159) |

### *1.9. Western-blot*

Myoblast C2C12 cells, treated with either (i) PBS + Tamoxifen (20 µM), (ii) ASO ctrl (2 µM), (iii) ASO ctrl (2 µM) + Tamoxifen (20 µM), (iv) ASO-Dnm2 (2 µM) or (v) ASO-Dnm2 (2 µM) + Tamoxifen (20 µM), were lysed in RIPA buffer (Thermo Scientific) supplemented with Halt protease inhibitor cocktail (Thermo Scientific). Protein concentration was determined with the BioRad DC protein Assay kit. Samples were diluted in RIPA buffer, NuPAGE LDS sample buffer and NuPAGE Sample Reducing Agent and denaturated at 70°C for 10 min. 5µg of protein was loaded, separated in 4-15% precast polyacrylamide gels by electrophoresis (BioRad) and transferred on nitrocellulose membrane for 7 min (Trans-Blot Turbo Transfer System, BioRad). Membranes were blocked for 5 min at room temperature in EveryBlot Blocking Buffer (Biorad) before an incubation for 1h at room temperature with primary mouse monoclonal antibodies against DNM2 (1/250), or GAPDH (1/1000) diluted in blocking buffer. Goat anti-mouse secondary antibody coupled to horseradish peroxidase (1/500 for GAPDH; 1/250 for DNM2) was incubated 1h at room temperature with membranes. Nitrocellulose membranes were then visualized with a Fusion FX imager (Vilbert).

### *1.10. Statistical analysis*

Statistical analysis was performed with the software GraphPad Prism 9.1.0 (GraphPad Software, Inc.). The tests performed are indicated in Figure legends.

2. RESULTS

### 2.1. Lifespan

See **Figure 1.**

*Mtm1*$^{-/y}$ mice injected with ASO-ctrl (control ASO) exhibited a low survival rate of 20% at 12 weeks old (which corresponds to the end of the study) due to disease progression.

By contrast, administration of ASO-Dnm2, tamoxifen or ASO-Dnm2 combined with Tamoxifen led to increased lifespan of *Mtm1*$^{-/y}$ mice with 60%, 70% and 70% survival rate at the end of the study, respectively.

These results show that ASO-Dnm2 alone, tamoxifen alone and combination of the invention improve the lifespan of treated mice, with the combination providing a similar improvement to ASO-Dnm2 or tamoxifen alone by the end of the study. Besides, the combination therapy at the chosen doses was not toxic, since no unexplained death was observed.

### 2.2. Body weight

See **Figure 2.**

The body weight of *Mtm1*$^{-/y}$ mice treated with ASO-ctrl plateaued at 15.4 ± 2.7 g (mean ± SD), while the body weight of WT mice reached 28.6 ± 2 g at 12 weeks of age.

By contrast, treatment of *Mtm1*$^{-/y}$ mice with ASO-Dnm2 and the combination therapy ASO-Dnm2 + Tam improved body weight gain to a maximum of 20.1 ± 2.2 g for ASO-Dnm2 and of 21.9 ± 2.3 for the combination, at 12 weeks.

Tamoxifen alone did not improve the body weight compared to *Mtm1*$^{-/y}$ ASO-ctrl (15.4 ± 2.7g vs 15.3 ± 0.4g).

Hence, these results show that tamoxifen alone does not improve body weight, in contrast to ASO-Dnm2 alone and combination of the invention. The latter seems to provide a slight (yet, apparent) better body weight gain than therapy with ASO-Dnm2 alone.

### 2.3. Hanging test

See **Figure 3.**

Whole-body strength of *Mtm1*$^{-/y}$ mice injected with ASO-ctrl was significantly impaired over time, and the mice could not perform the hanging test from 7 weeks of age, with a mean ± SD holding time of 11 ± 4.2 s at 12 weeks of age. By contrast, the WTcontrol mice were able to hold for the 60 s duration of the test.

Systemic injection of ASO-Dnm2 at 3 mg/kg in affected *Mtm1*$^{-/y}$ mice did not restore whole-body strength with a mean ± SD holding time of 5.3 ± 6.7 s at 12 weeks of age, as previously reported by Tasfaout et al. (Nat Commun, 2017; 8: 15661) for this low dose.

Compared to ASO-Dnm2, tamoxifen treatment alone improved the hanging ability, reaching a mean holding time of 35.3 ± 24.4 s at 12 weeks of age.

Surprisingly, despite the relatively low dose of ASO-Dnm2 administered and the lack of effect with this dose delivered alone, the combination ASO-Dnm2 + Tam provided the greatest improvement in muscular strength of *Mtm1*$^{-/y}$ mice with a stronger effect than tamoxifen alone (53.4 ± 7.4 s vs 35.3 ± 24.4 s at 12 weeks old). ASO-Dnm2 + Tam treatment even restored whole-body strength back to WT levels from 5 weeks of age.

These results demonstrate that, despite using a low dose of ASO-Dnm2 which does not seem to provide a beneficial effect on its own, the combination therapy according to the invention is capable to greatly improve whole-body strength in a synergistic manner.

### 2.4. Disease Severity Score (DSS)

See **Figure 4.**

All WT mice had a mean DSS close to zero. This DSS remained low at any age.

At 4 weeks of age and before treatment, the DSS of *Mtm1*$^{-/y}$ mice was comprised between 0.8 and 1.0. As expected, the DSS of these mice treated with ASO-ctrl increased rapidly due to hanging and walking difficulties, loss of body weight and increasing kyphosis severity, reaching a score of about 4 before death.

Systemic injection of ASO-Dnm2 alone at 3 mg/kg, or of tamoxifen alone, in affected *Mtm1*$^{-/y}$ mice did not really improve the DSS, reaching, at 12 weeks old, a mean score of 3.8 for the ASO-Dnm2 and of 3.3 for tamoxifen.

By contrast, the combination therapy ASO-Dnm2 + Tam in affected *Mtm1*$^{-/y}$ mice was surprisingly able to greatly reduce the DSS, reaching a mean score of 1.4 at 12 weeks old.

These results demonstrate that the combination therapy according to the invention is capable to greatly reduce the severity of the disease, in a synergistic manner.

***2.5. Expression of Dynamin 2 (RNA and protein)***

See **Figure 5.**

In C2C12 mouse myoblast cells, treatment with 2 μM of ASO-Dnm2 greatly reduced the expression of *Dnm2* at the mRNA level, by about 70% compared to PBS control or ASO-ctrl. In contrast, 20 μM of tamoxifen (with PBS control, or with ASO-ctrl), did not reduce *Dnm2* mRNA expression. The combination of ASO-Dnm2 and tamoxifen reduced *Dnm2* mRNA levels similarly to ASO-Dnm2 alone, by about 70%, demonstrating the lack of effect of tamoxifen on *Dnm2* mRNA expression.

At the protein level, treatment with 2 μM of ASO-Dnm2 greatly reduced the expression of *DNM2* by about 70%, compared to ASO-ctrl. In contrast, regardless of its dose (5, 10 or 20 μM Tam, with PBS control, or with ASO-ctrl), tamoxifen did not reduce DNM2 protein expression. The combination of ASO-Dnm2 and tamoxifen reduced DNM2 protein levels similarly to ASO-Dnm2 alone, by about 70%, demonstrating the lack of effect of tamoxifen on DNM2 protein expression.

3. <u>CONCLUSION</u>

The combination according to the invention, notably at low doses, provides unexpected superior benefits than either treatment alone, since it can improve not only the lifespan, but also body weight, can restore whole-body strength, and significantly reduce the Disease Severity Score (DSS).

**EXAMPLE 2**

An antisense oligonucleotide targeting *Dnm2* as shown in Figure 6 is co-administered with tamoxifen in patients suffering from a dystrophinopathy or a centronuclear myopathy.

**Claims**

1. A combination for use in the treatment of a myopathy selected from a dystrophinopathy or a centronuclear myopathy (CNM), the combination comprising:

(i) a dynamin 2 inhibitor; and
(ii) a compound of formula (I), or an analog or a metabolite thereof,

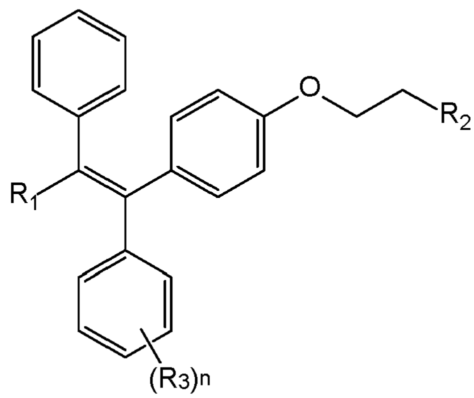

(I),

wherein

- $R_1$ is a radical selected from:

◦ a $(C_1-C_6)$alkyl group optionally substituted by a halogen,
◦ a hydrogen, or
◦ a halogen;

• $R_2$ is a radical selected from:

◦ a $-NR_4R_5$ group with $R_4$ and $R_5$ being independently a hydrogen, a $(C_1-C_6)$alkyl group said $(C_1-C_6)$alkyl group being optionally substituted by a $-CF_3$ or a $-OCF_3$ group, or $R_4$ and $R_5$ form together a heterocycloalkyl, or
◦ a $-OR_6$ group with $R_6$ being a H or a $(C_1-C_6)$alkyl group substituted by a hydroxy;

• n is 0, 1, or 2; and/or
• $R_3$ is a radical selected from:

◦ a hydrogen,
◦ a hydroxy, or
◦ a halogen, preferably a iodine;

or a pharmaceutically acceptable salt, solvate, oxide, or stereoisomer thereof.

**2.** The combination for use according to claim 1, wherein the dystrophinopathy is Duchenne muscular dystrophy or Becker muscular dystrophy, preferably Duchenne muscular dystrophy.

**3.** The combination for use according to claim 1, wherein the centronuclear myopathy is X-linked centronuclear myopathy, autosomal recessive centronuclear myopathy or autosomal dominant centronuclear myopathy, preferably X-linked centronuclear myopathy.

**4.** The combination for use according to any one of the precedent claims, wherein the dynamin 2 inhibitor is selected from the group consisting of a nucleic acid molecule interfering specifically with dynamin 2 expression, an antibody directed against dynamin 2, a genome editing system comprising a nuclease engineered to target the *DNM2* gene, and a small molecule inhibiting the dynamin 2 activity, expression or function, and any combinations thereof.

**5.** The combination for use according to claim 4, wherein the nucleic acid molecule interfering specifically with dynamin 2 expression is an antisense nucleic acid, an RNAi nucleic acid, or a ribozyme interfering specifically with dynamin 2 expression.

**6.** The combination for use according to claim 4 or 5, wherein the nucleic acid molecule interfering specifically with dynamin 2 expression is complementary to at least one intron or the 3'UTR of dynamin 2 pre-mRNA, preferably to at least intron 12, intron 13, intron 11, intron 1, intron 14 of dynamin 2 pre-mRNA.

**7.** The combination for use according to claim 6, wherein the nucleic acid molecule interfering specifically with dynamin 2 expression is an antisense nucleic acid comprising or consisting of at least 8 contiguous nucleobases of any one of SEQ ID NO: 1 to 3135, preferably is antisense nucleic acid comprising or consisting of any one of the sequences of SEQ ID NO: 1 to 3135.

**8.** The combination for use according to claim 4 or 5, wherein the nucleic acid molecule interfering specifically with dynamin 2 expression induces exon-skipping within a dynamin 2 pre-mRNA, preferably of at least exon 2 and/or 8 of dynamin 2 pre-mRNA.

**9.** The combination for use according to claim 8, wherein the nucleic acid molecule interfering specifically with dynamin 2 expression is an antisense nucleic acid comprising or consisting of the sequence SEQ ID NO: 3136 or SEQ ID NO: 3137.

**10.** The combination for use according to claim 4 or 5, wherein the nucleic acid molecule interfering specifically with dynamin 2 expression is complementary to at least one exon of dynamin 2 mRNA, preferably to at least exon 1, 4, 5, 12b, 13, 15, 17 and/or 21 of dynamin 2 mRNA.

**11.** The combination for use according to claim 10, wherein the nucleic acid molecule interfering specifically with dynamin

2 expression is an RNAi nucleic acid comprising or consisting of any one of the sequences SEQ ID NO: 3138 to SEQ ID NO: 3151.

**12.** The combination for use according to any one of the preceding claims, wherein the compound of formula (I) is such that:

- $R_1$ is a radical selected from:
  - a $(C_1\text{-}C_6)$alkyl group, preferably an ethyl group, optionally substituted by a halogen, preferably a chlorine; or
  - a halogen, preferably a chlorine;
- $R_2$ is a radical selected from:
  - a $-NR_4R_5$ group with $R_4$ and $R_5$ being independently a hydrogen, a $(C_1\text{-}C_6)$alkyl group, preferably a methyl or an ethyl group, or $R_4$ and $R_5$ form together a pyrrolidinyl; or
  - a $-OR_6$ group with $R_6$ being a H or a $(C_1\text{-}C_6)$alkyl group, preferably an ethyl group, substituted by a hydroxy;
- n is 0 or 1 ; and/or
- $R_3$ is a radical selected from: a hydrogen, a hydroxy, or a halogen, preferably a iodine.

**13.** The combination for use according to any one of the preceding claims, wherein the compound of formula (I) is such that $R_1$ is an ethyl group, $R_2$ is a $NR_4R_5$ group with $R_4$ and $R_5$ being independently a methyl group, and n is 0.

**14.** The combination for use according to any one of the preceding claims, wherein the analogs thereof are selected from the group consisting of:

- 6-(2,4-dichlorophenyl)-5-[4-[(3S)-1-(3-fluoropropyl)pyrrolidin-3-yl]oxyphenyl]-8,9-dihydro-7H-benzo[7]annulene-2-carboxylic acid;
- (5R,6S)-6-phenyl-5-[4-(2-pyrrolidin-1-ylethoxy)phenyl]-5,6,7,8-tetrahydronaphthalen-2-ol;
- [6-hydroxy-2-(4-hydroxyphenyl)-1-benzothiophen-3-yl]-[4-(2-piperidin-1-ylethoxy)phenyl]methanone;
- 1-[[4-[2-(azepan-1-yl)ethoxy]phenyl]methyl]-2-(4-hydroxyphenyl)-3-methylindol-5-ol;
- (E)-4-[2-[4-[(E)-1-(1H-indazol-5-yl)-2-phenylbut-1enyl]phenoxy]ethylamino]-N,N-dimethylbut-2-enamide; and
- (E)-3-[4-[(E)-2-(2-chloro-4-fluorophenyl)-1-(1H-indazol-5-yl)but-1-enyl]phenyl]prop-2-enoic acid.

**15.** (i) A dynamin 2 inhibitor and (ii) a compound of formula (I) or an analog or a metabolite thereof, or a pharmaceutically acceptable salt, solvate, oxide, or stereoisomer thereof, as defined in any one of the preceding claims, as a combined preparation for simultaneous, separate or sequential use in the treatment of a myopathy as defined in any one of claims 1 to 3.

**16.** A pharmaceutical composition comprising (i) a dynamin 2 inhibitor and (ii) a compound of formula (I) or an analog or a metabolite thereof, or a pharmaceutically acceptable salt, solvate, oxide, or stereoisomer thereof, as defined in any one of the preceding claims, and optionally (iii) a pharmaceutically acceptable excipient.

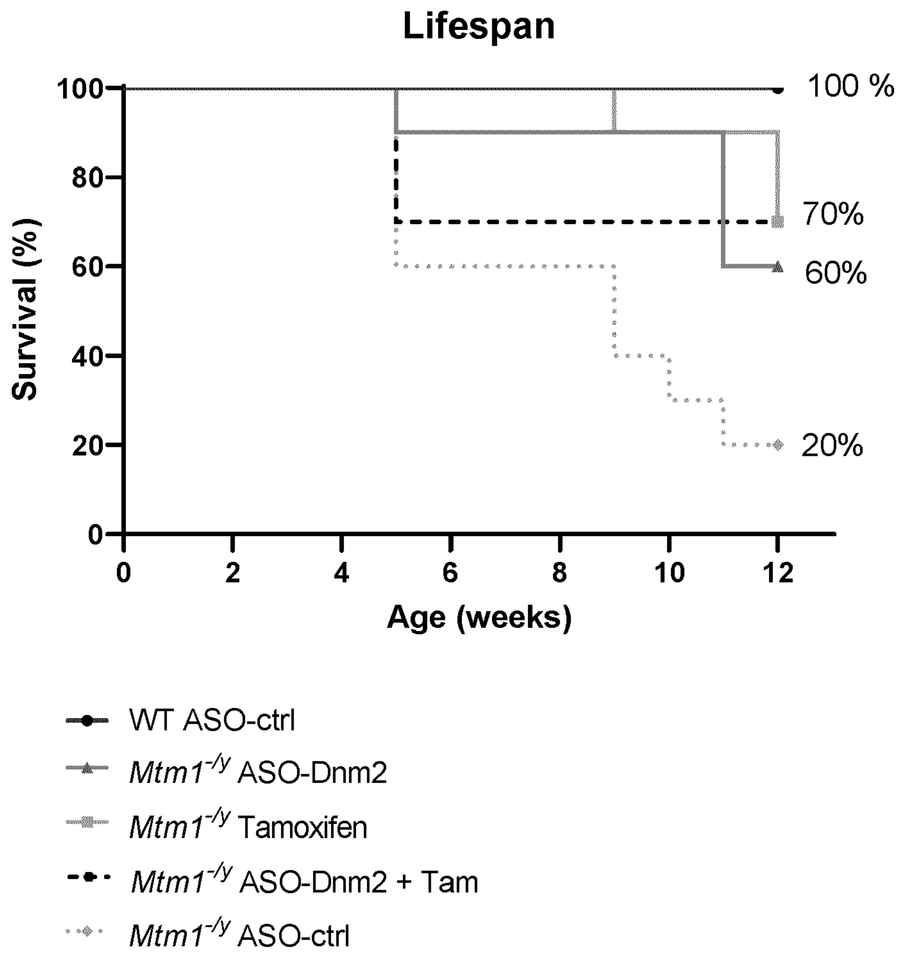
Lifespan
Survival (%)
100
80
60
40
20
0
0
2
4
6
8
10
12
Age (weeks)
100 %
70%
60%
20%
WT ASO-ctrl
Mtm1-/y ASO-Dnm2
Mtm1-/y Tamoxifen
Mtm1-/y ASO-Dnm2 + Tam
Mtm1-/y ASO-ctrl

FIGURE 1

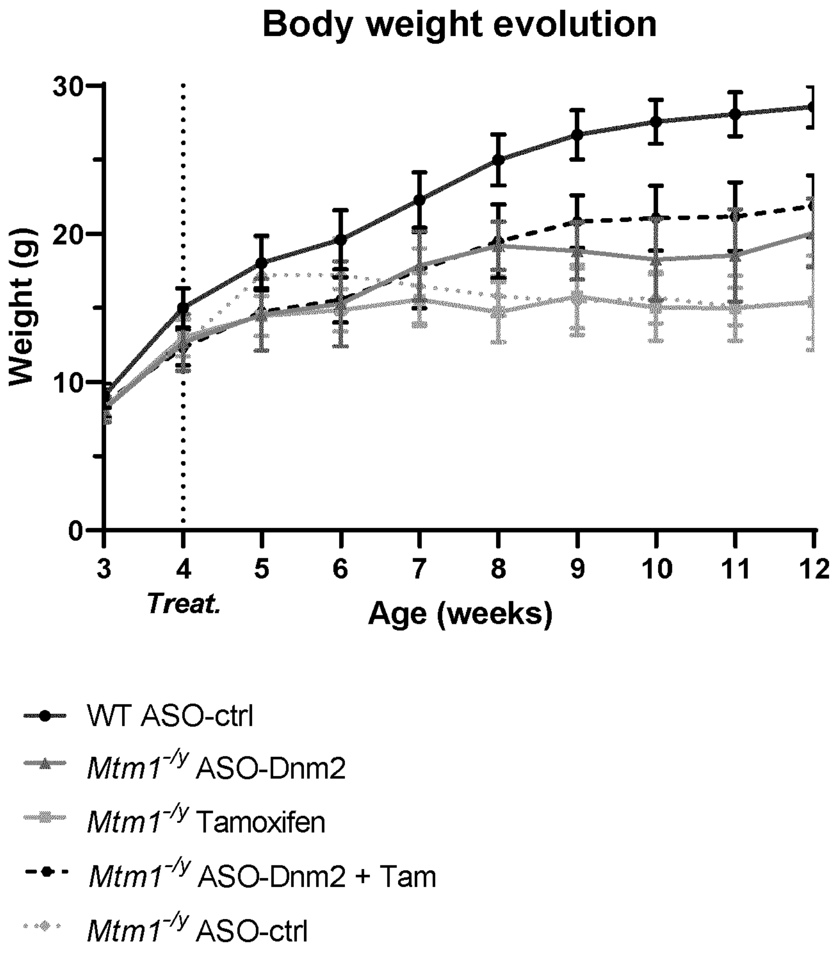
Body weight evolution
Weight (g)
30
20
10
0
3
4
5
6
7
8
9
10
11
12
Treat.
Age (weeks)
WT ASO-ctrl
Mtm1-/y ASO-Dnm2
Mtm1-/y Tamoxifen
Mtm1-/y ASO-Dnm2 + Tam
Mtm1-/y ASO-ctrl

FIGURE 2A

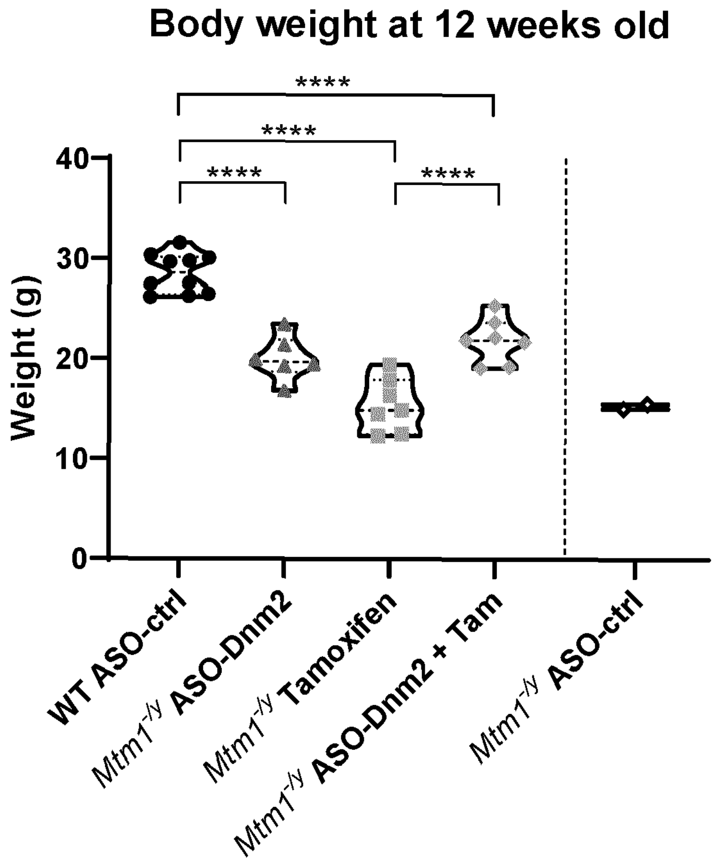

Body weight at 12 weeks old
****
****
****
****
Weight (g)
40
30
20
10
0
WT ASO-ctrl
Mtm1-/y ASO-Dnm2
Mtm1-/y Tamoxifen
Mtm1-/y ASO-Dnm2 + Tam
Mtm1-/y ASO-ctrl


FIGURE 2B

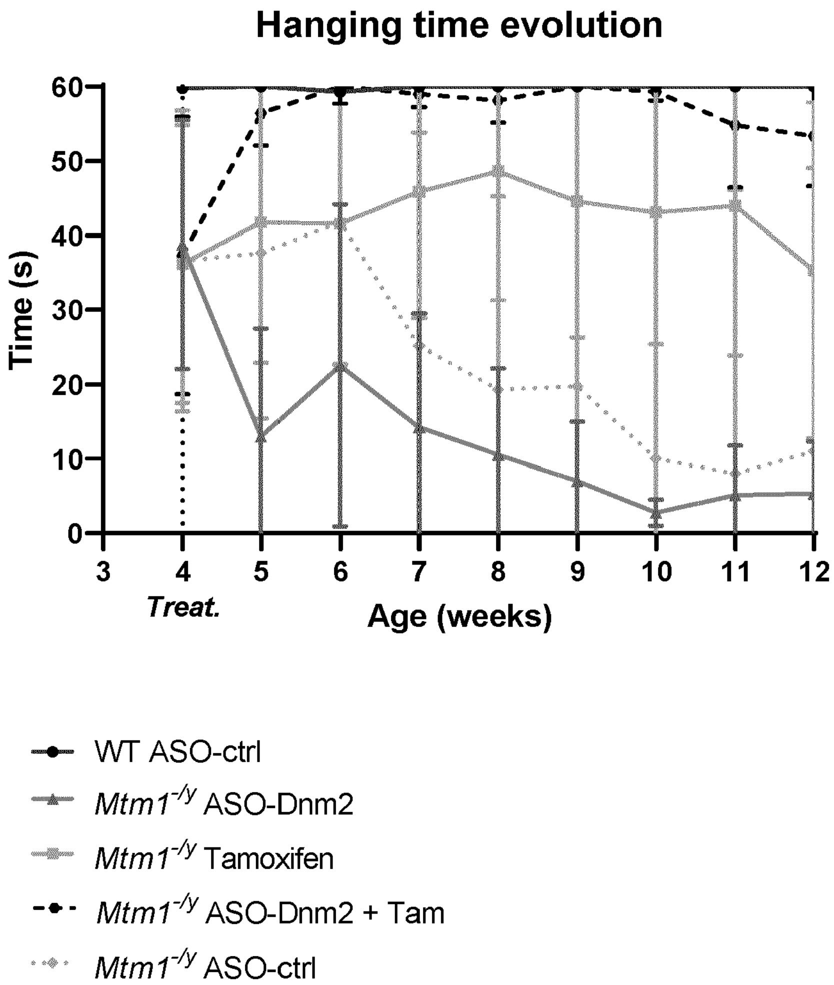
Hanging time evolution
Time (s)
60
50
40
30
20
10
0
3
4
5
6
7
8
9
10
11
12
Treat.
Age (weeks)
WT ASO-ctrl
Mtm1-/y ASO-Dnm2
Mtm1-/y Tamoxifen
Mtm1-/y ASO-Dnm2 + Tam
Mtm1-/y ASO-ctrl

FIGURE 3A

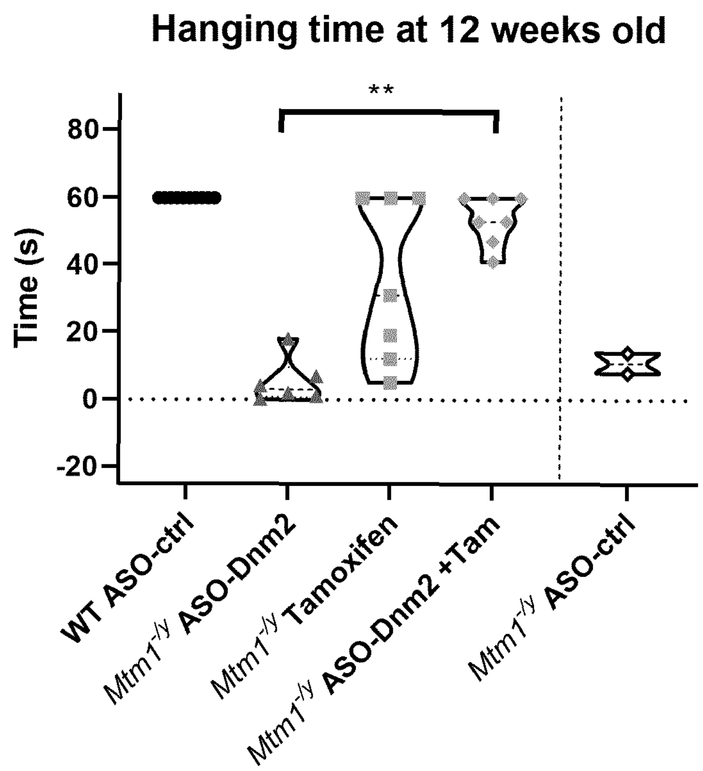
Hanging time at 12 weeks old
**
80
60
40
20
0
-20
Time (s)
WT ASO-ctrl
Mtm1-/y ASO-Dnm2
Mtm1-/y Tamoxifen
Mtm1-/y ASO-Dnm2 +Tam
Mtm1-/y ASO-ctrl

FIGURE 3B

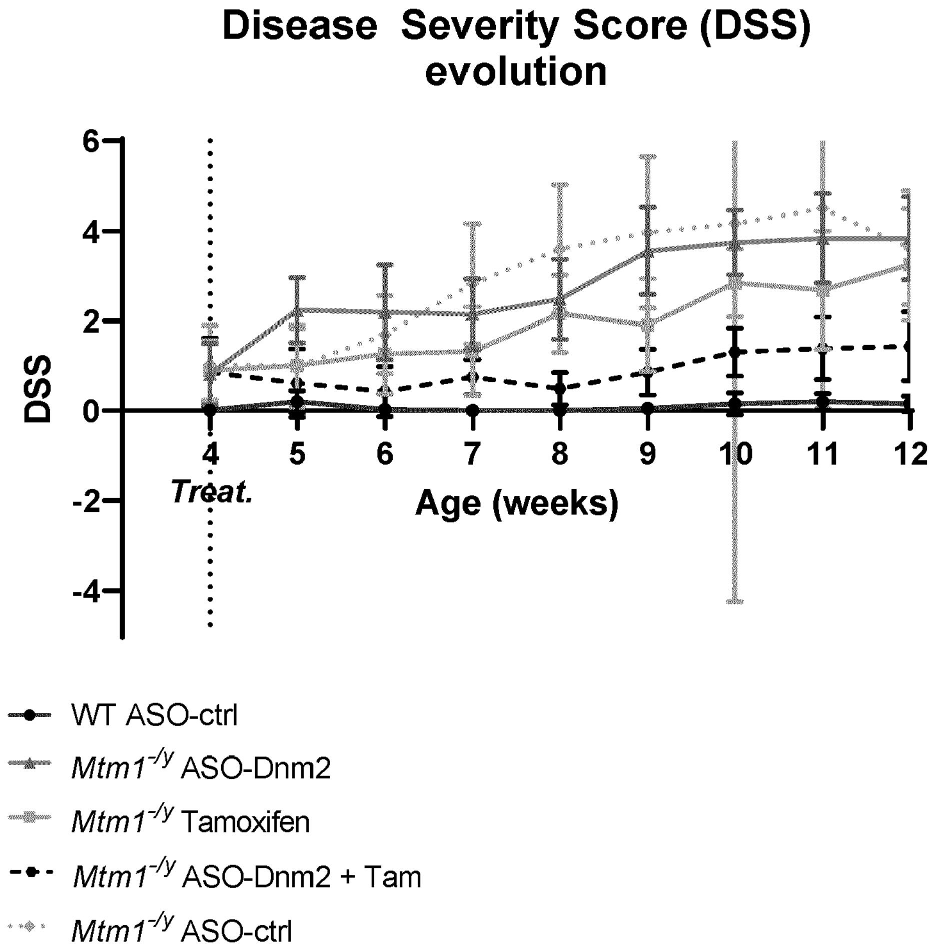
Disease Severity Score (DSS) evolution
DSS
6
4
2
0
-2
-4
4
5
6
7
8
9
10
11
12
Treat.
Age (weeks)
WT ASO-ctrl
Mtm1-/y ASO-Dnm2
Mtm1-/y Tamoxifen
Mtm1-/y ASO-Dnm2 + Tam
Mtm1-/y ASO-ctrl

FIGURE 4A

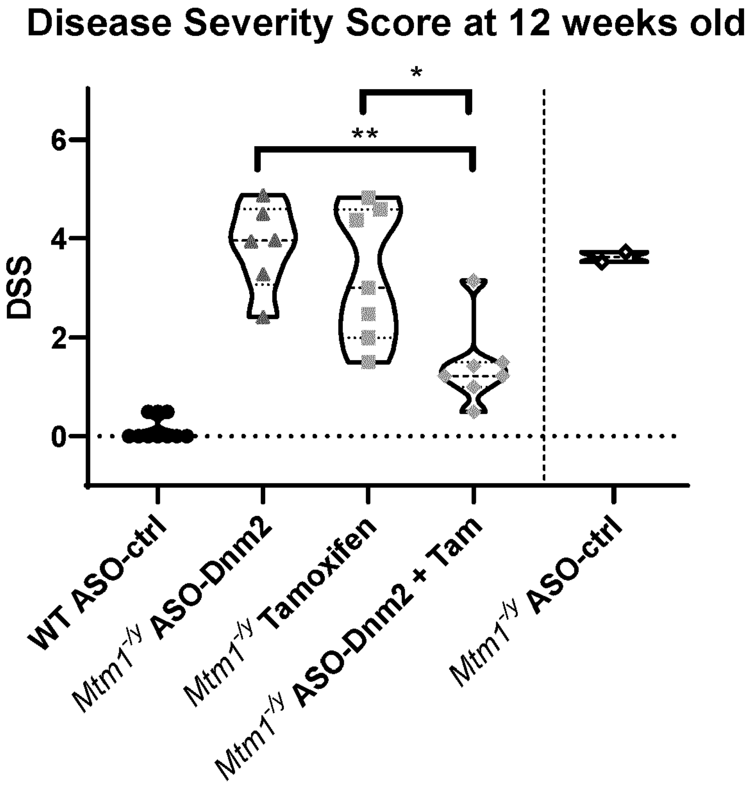
Disease Severity Score at 12 weeks old
*
**
DSS
6
4
2
0
WT ASO-ctrl
Mtm1-/y ASO-Dnm2
Mtm1-/y Tamoxifen
Mtm1-/y ASO-Dnm2 + Tam
Mtm1-/y ASO-ctrl

FIGURE 4B

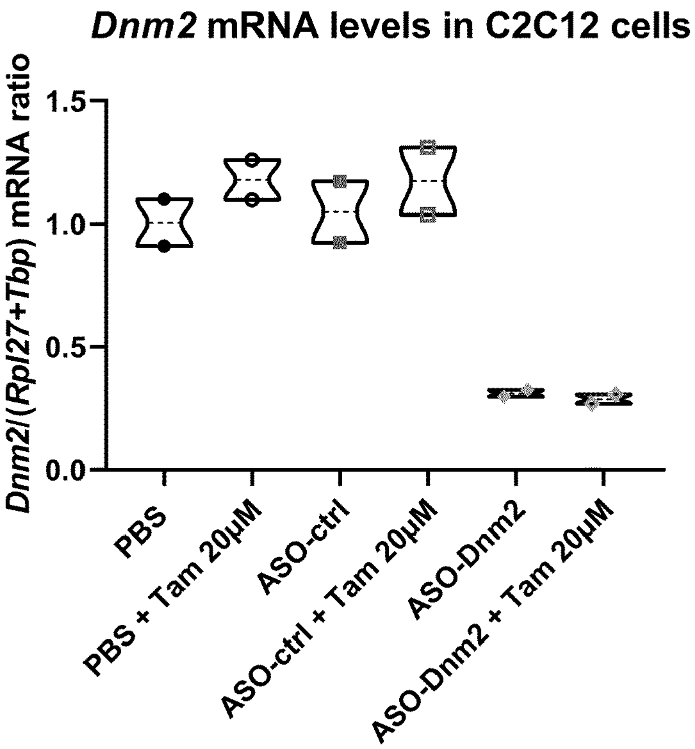
*Dnm2* mRNA levels in C2C12 cells
*Dnm2*/(*Rpl27*+*Tbp*) mRNA ratio
1.5
1.0
0.5
0.0
PBS
PBS + Tam 20µM
ASO-ctrl
ASO-ctrl + Tam 20µM
ASO-Dnm2
ASO-Dnm2 + Tam 20µM

FIGURE 5A

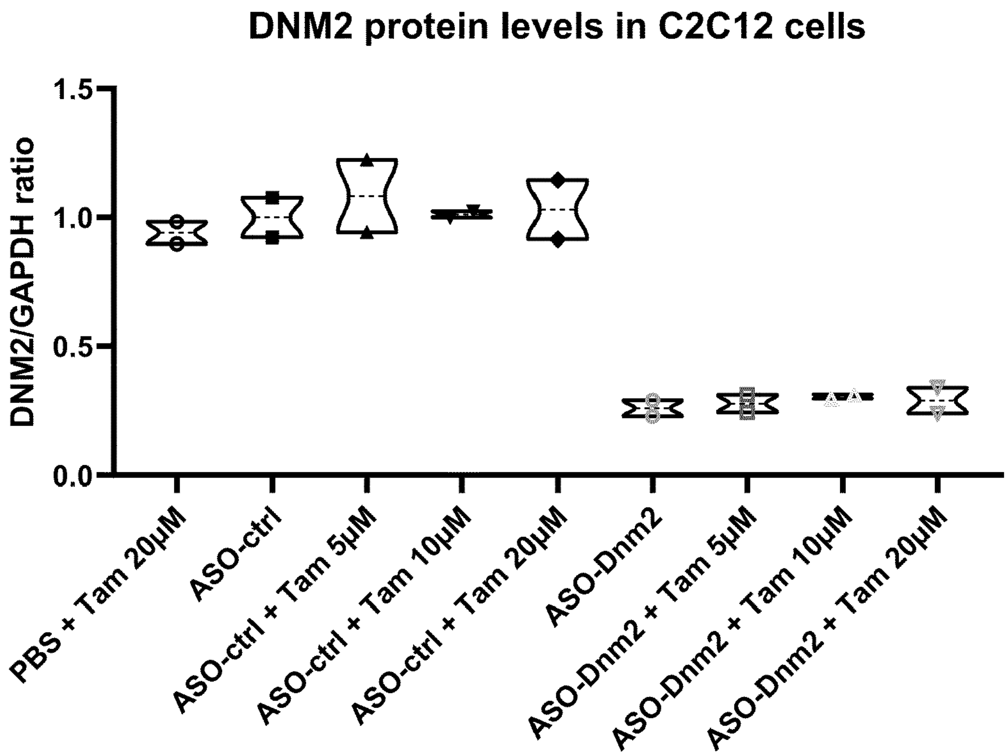
DNM2 protein levels in C2C12 cells
DNM2/GAPDH ratio
1.5
1.0
0.5
0.0
PBS + Tam 20µM
ASO-ctrl
ASO-ctrl + Tam 5µM
ASO-ctrl + Tam 10µM
ASO-ctrl + Tam 20µM
ASO-Dnm2
ASO-Dnm2 + Tam 5µM
ASO-Dnm2 + Tam 10µM
ASO-Dnm2 + Tam 20µM

FIGURE 5B

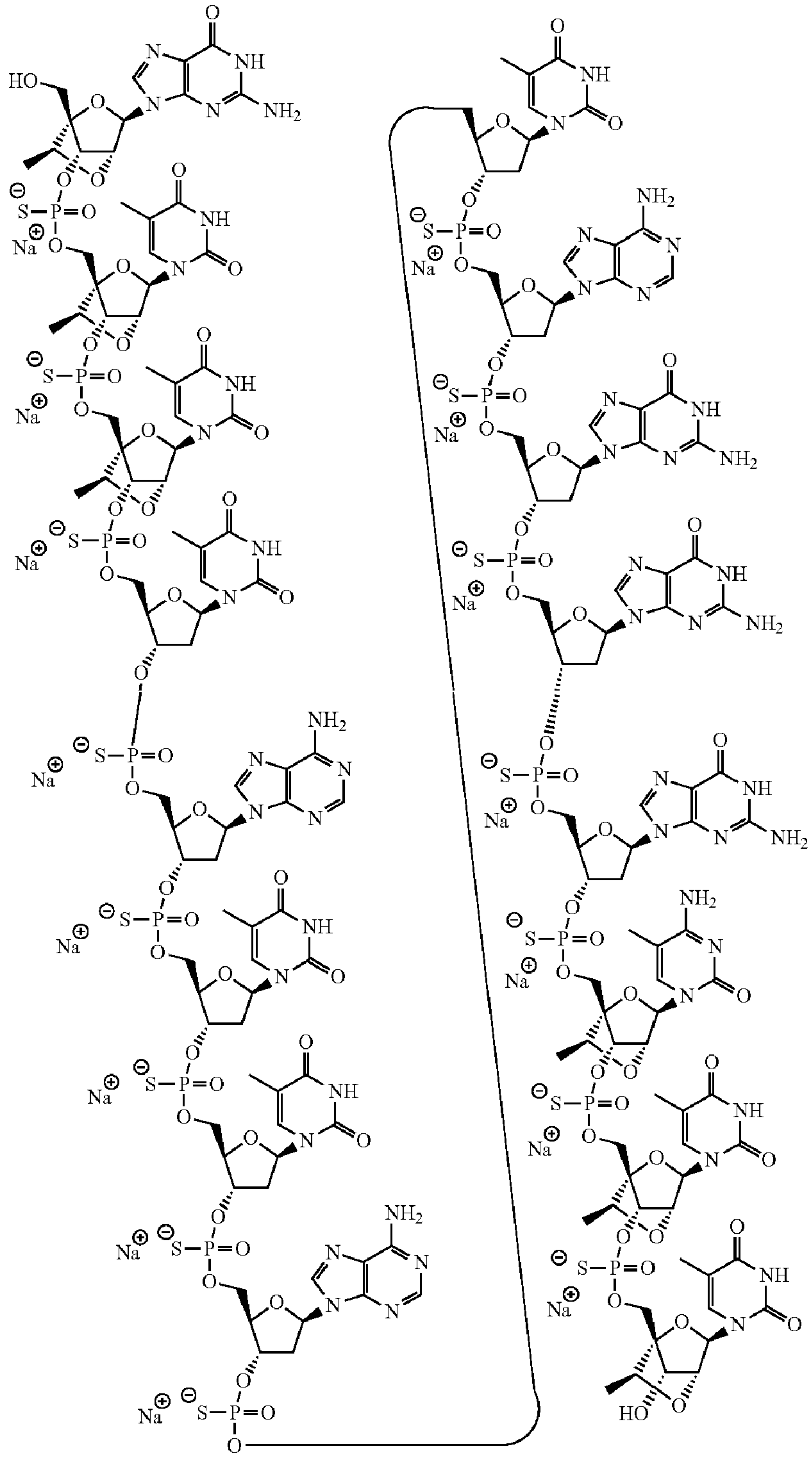

(SEQ ID NO: 3129)

# FIGURE 6

Europäisches Patentamt
European Patent Office
Office européen des brevets

# EUROPEAN SEARCH REPORT

Application Number
EP 22 30 6112

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DOWLING JAMES J ET AL: "Molecular and cellular basis of genetically inherited skeletal muscle disorders", NATURE REVIEWS MOLECULAR CELL BIOLOGY, NATURE PUBLISHING GROUP UK, LONDON, vol. 22, no. 11, 13 July 2021 (2021-07-13), pages 713-732, XP037600428, ISSN: 1471-0072, DOI: 10.1038/S41580-021-00389-Z [retrieved on 2021-07-13] * paragraph bridging page 721 and 722 * ----- | 1-16 | INV. A61K31/138 A61K45/06 C12N15/113 A61P21/00 |
| Y | DJEDDI SARAH ET AL: "Multi-omics comparisons of different forms of centronuclear myopathies and the effects of several therapeutic strategies", MOLECULAR THERAPY, vol. 29, no. 8, 1 August 2021 (2021-08-01), pages 2514-2534, XP093011279, US ISSN: 1525-0016, DOI: 10.1016/j.ymthe.2021.04.033 * abstract; page 20-22, lines 463-516 * ----- -/-- | 1-16 | |
| | | | TECHNICAL FIELDS SEARCHED (IPC) |
| | | | A61K A61P C12N |

The present search report has been drawn up for all claims

| Place of search | Date of completion of the search | Examiner |
|---|---|---|
| Munich | 3 January 2023 | Borst, Markus |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 22 30 6112

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GAYI ELINAM ET AL: "Tamoxifen prolongs survival and alleviates symptoms in mice with fatal X-linked myotubular myopathy", NATURE COMMUNICATIONS, vol. 9, no. 1, 19 November 2018 (2018-11-19), pages 1-14, XP093011153, DOI: 10.1038/s41467-018-07058-4 Retrieved from the Internet: URL:https://www.ncbi.nlm.nih.gov/pmc/articles/PMC6243013/pdf/41467_2018_Article_7058.pdf> | 1-3, 12-16 | |
| Y | * abstract; page 2, left hand column, 2nd paragraph; page 8, right hand column, last full paragraph * | 1-16 | |
| | ----- | | |
| Y,D | WO 2018/189208 A1 (GENETHON [FR]; INST NAT SANTE RECH MED [FR] ET AL.) 18 October 2018 (2018-10-18) * page 4, line 15-32 * | 1-16 | |
| | ----- | | TECHNICAL FIELDS SEARCHED (IPC) |
| Y | BELINDA S. COWLING ET AL: "Reducing dynamin 2 expression rescues X-linked centronuclear myopathy", JOURNAL OF CLINICAL INVESTIGATION, vol. 124, no. 3, 3 March 2014 (2014-03-03), pages 1350-1363, XP055105947, ISSN: 0021-9738, DOI: 10.1172/JCI71206 * abstract * | 1-16 | |
| | ----- | | |

The present search report has been drawn up for all claims

1

| Place of search | Date of completion of the search | Examiner |
|---|---|---|
| Munich | 3 January 2023 | Borst, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 30 6112

This annex lists the patent family members relating to the patent documents cited in the above-mentioned European search report. The members are as contained in the European Patent Office EDP file on
The European Patent Office is in no way liable for these particulars which are merely given for the purpose of information.

03-01-2023

| Patent document cited in search report | Publication date | Patent family member(s) | Publication date |
| --- | --- | --- | --- |
| WO 2018189208 A1 | 18-10-2018 | EP 3610016 A1 | 19-02-2020 |
| | | US 2021095291 A1 | 01-04-2021 |
| | | WO 2018189208 A1 | 18-10-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015055859 A1
- WO 2016170162 A1
- WO 2015055859 A
- WO 201800010 A
- WO 2018189208 A
- WO 2019140452 A
- WO 2020028844 A
- WO 9932619 A
- WO 2018100010 A
- GB 1064629 A
- EP 0168175 A1


### Non-patent literature cited in the description


- **GÓMEZ-OCA et al.** *Int J Mol Sci.,* 2021, vol. 22 (21), 11377
- **AL ZAIDI et al.** *Pediatr Neurol.,* 2014, vol. 51 (5), 607-618
- **COWLING et al.** *J Clin Invest.,* 2014, vol. 124 (3), 1350-63
- **TASFAOUT et al.** *Nat Commun,* 2017, vol. 8, 15661
- **BUONO et al.** *Proc Natl Acad Sci U S A,* 2018, vol. 115 (43), 11066-11071
- **TROCHET et al.** *EMBO Mol Med.,* 2018, vol. 10 (2), 239-253
- **JUNGBLUTH et al.** *Orphanet J Rare Dis,* 2008, vol. 3, 26
- **LAPORTE et al.** *Nature Genetics,* 1996, vol. 13 (2), 175-182
- **BITOUN et al.** *Nature Genetics,* 2005, vol. 37 (11), 1207-1209
- **BOHM et al.** *Brain,* 2014, vol. 137, 3160-3170
- **KERST et al.** *Neuromuscul Disord.,* 2000, vol. 10, 572-577
- **TOSCH et al.** *Hum Mol Genet.,* 2006, vol. 15 (21), 3098-3106
- **MAJCZENKO et al.** *Am. J. Hum. Genet.,* 2012, vol. 91, 365-371
- **NICOT et al.** *Nature Genetics,* 2007, vol. 39 (9), 1134-1139
- **VALSI et al.** *Brain,* 2017, vol. 140, 37-48
- **MUNTONI et al.** *Lancet Neurol,* 2003, vol. 2, 731-740
- **TUFFERY-GIRAUD et al.** *Hum Mutat.,* 2009, vol. 30 (6), 934-45
- **BLADEN et al.** *Hum Mutat.,* 2015, vol. 36 (4), 395-402
- **MACIA E. et al.** *Developmental cell,* 2006, vol. 10, 839-850
- **MCCLUSKEY et al.** *Traffic,* 2013, vol. 14 (12), 1272-89
- **MCGEACHIE et al.** *ACS Chem Biol,* 2013, vol. 8 (7), 1507-18
- **WANG et al.** *J Biol Chem,* 2010, vol. 285 (30), 22753-7
- **KENNISTON ; LEMMON.** *EMBO J,* 2010, vol. 29 (18), 3054-67
- **BUONO et al.** *Proc Natl Acad Sci USA,* 2018, vol. 115 (43), 11066-11071
- **ECKSTEIN F.** *Antisense and Nucleic Acid Drug Development,* 2000, vol. 10 (2), 1117-221
- **LEE et al.** *J Cardiovasc Transl Res.,* 2013, vol. 6 (6), 969-80
- **DEVOS et al.** *Neurotherapeutics,* 2013, vol. 10 (3), 486-972013
- **TINELLI et al.** *Hum Mol Genet.,* 2013, vol. 22 (21), 4417-29
- **SOHAIL et al.** Gene Silencing by RNA Interference: Technology and Application. 2004
- **WANG et al.** *Pharm Res,* 2011, vol. 28, 2983-2995
- **BERNSTEIN et al.** *Nature,* 2001, vol. 409 (6818), 363-6
- **ZAMORE et al.** *Cell,* 2000, vol. 101 (l), 25-33
- **ELBASHIR et al.** *Genes Dev.,* 2001, vol. 15 (2), 188-200
- **ELBASHIR et al.** *EMBO J,* 2001, vol. 20 (23), 6877-88
- **FANNING ; SYMONDS.** RNA Towards Medicine (Handbook of Experimental Pharmacology). Springer, 2006, 289-303
- **MALI et al.** *Nature Methods,* 2013, vol. 10 (10), 957-63
- **JANEWAY et al.** Immunobiology. Garland publishing, 2001
- **AN et al.** Therapeutic Monoclonal Antibodies: From Bench to Clinic. Wiley, 2009
- **SKRLEC et al.** *Trends in Biotechnology,* 2015, vol. 33 (7), 408-41

- **HARLOW, E. ; LANE, D.** Antibodies: A Laboratory Manual. Cold Spring Harbor Laboratory, 1988
- **HARPER et al.** *Trends Cell Biol.,* February 2013, vol. 23 (2), 90-101
- *CHEMICAL ABSTRACTS,* 304448-55-3
- *CHEMICAL ABSTRACTS,* 1256493-34-1
- *CHEMICAL ABSTRACTS,* 1119-97-7
- **ORCHIN et al.** The Vocabulary of Organic Chemistry. John Wiley and Sons, Inc, 1980, 126
- **BEDFORD et al.** *Nature,* 1996, vol. 212
- **TANDON et al.** *Asian Journal or Organic Chemistry,* 2020
- Remington: The Science and Practice of Pharmacy. Lippincott Williams & Wilkins, 2000
- Encyclopedia of Pharmaceutical Technology. Marcel Dekker, 1988
- **SETH et al.** *Nucleic Acids Symp Ser (Oxf),* 2008, vol. 52, 553-554
- **BUJ-BELLO et al.** *Proc Natl Acad Sci USA,* 2002, vol. 99, 15060-15065
- **AL-QUSAIRI.** *Proc Natl Acad Sci USA,* 2009, vol. 106, 18763-18768
- **BUONO et al.** *Dis Model Mech.,* 2022